# EUROPEAN PATENT SPECIFICATION

(11) **EP 1 931 656 B1**
(45) Date of publication and mention of the grant of the patent: **09.02.2011**
(21) Application number: 06825333.5
(22) Date of filing: 28.09.2006
(51) Int. Cl.: C07D 405/12, C07D 471/04, C07K 5/06, A61K 31/4427, A61K 31/4375, A61K 38/05, A61P 25/28

(54) **NOVEL DRUGS FOR DEMENTIA**
NEUE ARZNEIMITTEL GEGEN DEMENZ
NOUVEAUX MEDICAMENTS CONTRE LA DEMENCE

(30) Priority: 28.09.2005 US 722066 P; 10.02.2006 US 772631 P
(43) Date of publication of application: 18.06.2008
(73) Proprietor: Ternansky, Robert, San Diego, CA 92130 (US)
(72) Inventor: Ternansky, Robert, San Diego, CA 92130 (US)
(74) Representative: Dunleavy, Kevin James
(86) International application number: PCT/US2006/038387
(87) International publication number: WO 2007/038772

(56) References cited:
- EP-A- 0 580 161
- US-A- 4 942 165
- KEN-ICHIRI KUWAKO ET. AL.: "Activation of calpain in cultured neurons overexpressing Alzheimer amyloid precursor protein." MOLECULAR BRAIN RESEARCH, vol. 107, 2002, pages 166-175, XP002419601
- QIAO-XIN LI ET. AL.: "Proteolytic Processing of Alzheimers Disease betaA4 Amyloid Precursor Protein in Human Platelets" JOURNAL OF BIOLOGICAL CHEMISTRY, vol. 270, no. 23, 1995, pages 14140-14147, XP000929629
- V. HOOK ET. AL: "beta-Amyloid peptide in regulated secretory vesicles of chromaffin cells: evidence for multiple cysteine proteolytic activities in distinct pathways for beta-secretase activity in chromaffin vesicles." JOURNAL OF NEUROCHEMISTRY, vol. 81, no. 2, 2002, pages 237-256, XP002419602 cited in the application
- Z. HUANG ET. AL.: "Ester and Amide Derivatives of E64c as Inhibitors of Platelet Calpains" JOURNAL OF MEDICINAL CHEMISTRY, vol. 35, 1992, pages 2048-2054, XP000644309
- E. POP ET. AL.: "Brain-Specific Chemical Delivery Systems for beta-Lactam Antibiotics. Synthesis and Properties of Some Dihydropyridine and Dihydroisoquinoline Derivatives of Benzylpenicillin." JOURNAL OF MEDICINAL CHEMISTRY, vol. 32, 1989, pages 1774-1781, XP002419603 cited in the application
- WHEI-MEI LU ET. AL.: "Brain-Specific Chemical Delivery Systems for beta-Lactam Antibiotics. In vitro and In Vivo Studies of Some Dihydropyridine and Dihydroisoquinoline Derivatives of Benzylpenicillin in Rats." JOURNAL OF MEDICINAL CHEMISTRY, vol. 32, 1989, pages 1782-1788, XP002419604 cited in the application

## Description

This invention was made with United States Federal government support under grant number 2 R44 AG18044, awarded by the National Institutes of Health the Federal government may have certain rights in this invention.

### TECHINCAL FIELD

This invention relates generally to chemistry, medicinal chemistry, enzymology, biochemistry, molecular and cellular biology, genetics, pharmacology, pharmacy, neurobiology, medicine, and drug therapy In particular, the present invention relates to novel prodrugs, pharmaceutical compositions containing them and methods of making and using them. Specifically, the invention includes prodrugs having improved properties for affecting dementia.

### BACKGROUND

Proteolytic enzymes catalyse the hydrolytic cleavage of peptide bonds In proteins and peptides. They can be classified as exopeptidases and endopeptidases (called proteinases or proteases) that are further subdivided into protease classes, the serine-, aspartic-, metallo- and cysteine proteases. Cysteine proteases are found in viruses and prokaryotes as well as plants and mammals, including man.

Cysteine proteases have a common catalytic mechanism arising from the presence of the catalytic amino acids cysteine and histidine in the active site of the enzyme, the site where the peptide bond is cleaved. Cleavage involves a nucleophilic cysteine thiol in a catalytic triad The first step is deprotonation of a thiol in the enzyme's active site by an adjacent amino acid with a basic side chain, usually a histidine residue. The next step is nucleophilic attack by the deprotonated cysteine's anionic sulfur on the substrate carbonyl carbon. In this step, a fragment of the substrate is released with an amine terminus, the histidine residue in the protease is restored to its deprotonated form, and a thioester intermediate linking the new carboxy-terminus of the substrate to the cysteine thiol is formed. The thioester bond is subsequently hydrolyzed to generate a carboxylic acid moeity on the remaining substrate fragment, while regenerating the free enzyme

Examples of cysteine proteases include the cathepsins, caspases, calpains and papain. Cathepsins are a large family that are distinguished by their substrate specificities and cathepsin B enzyme In the cathepsin class.

Cysteine proteases, cathepsins, and cathepsin B, specifically, are known to be involved in neurological pathologies. In particular, these enzymes are known to be important In the development of dementia, especially dementia due to Alzheimer's disease (AD) and vascular dementia (VD).

Huang, Z, et al, "Ester and Amide Derivatives of E64c as inhibitors of Platelet Calpains," J. of Medicinal Chemisry, Vol. 35(1992), pp 2048-54 tested the calpain inhibitor activity in vitro in lysed and intact cells for esters and amides of E64c, demonstrating that esters were as effective as E64c but that amides were not as effective as E64c in intact cells. EP-A-580 161 determined calpain inhibiting activity of E64c in vitro using m-calpain extracted from rat brains. U.S. Patent no 4,942,165 discloses certain lipophilic choline compounds which are said to be delivered specifically to the brain for Alzheimer's treatment though no working example of any kind are provided. Many cysteine protease, cathepsin protease and cathepsin B inhibitors are known in the art However, prodrug forms are needed having improved pharmacological and toxicological properties. In particular, new prodrugs are needed having superior properties for treating neurological pathologies, especially dementia. The present invention provides these prodrugs, compositions and method of using the same to treat dementia.

### SUMMARY

The invention is directed to novel prodrugs containing a chemical delivery system (CDS) moiety and a cysteine protease inhibitor moiety T he CDS moiety targets the cysteine protease inhibitor to the brain or central nervous system. The cysteine protease inhibitor moiety is inactive in the prodrug but active upon release Cysteine protease inhibitors are useful for treating dementia, Alzheimer's disease (AD) and vascular dementia (VD) and, thus, CDS prodrugs of the invention that target the brain and central nervous system offer significant advantages for treating those conditions and diseases. A preferred CDS prodrug contains a dihydrotrigoneline CDS moiety and an epoxysuccinyl peptide cysteine protease inhibitor moiety The invention includes pharmaceutical compositions containing a CDS prodrug and methods of using such compositions to treat those conditions and diseases

### BRIEF DESCRIPTION OF THE DRAWINGS

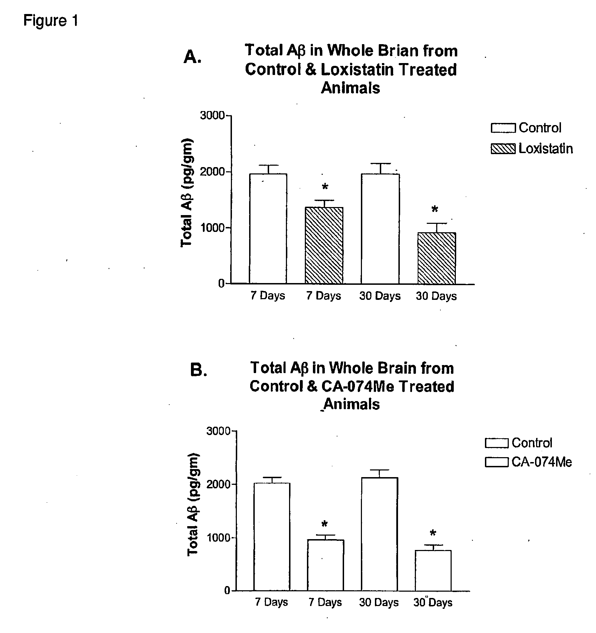

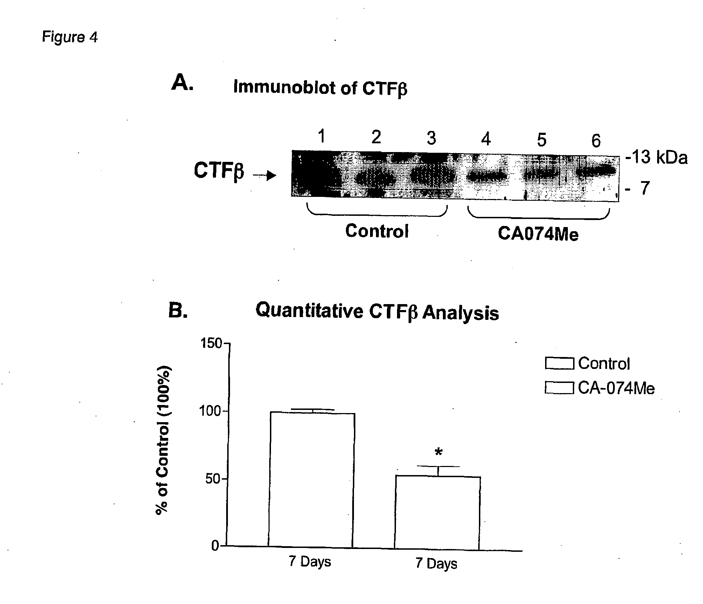

**Figure 1****. E64d or CA074Me reduce inAβ in brain *in vivo***. E64d (also known as loxistatin) or CA074Me were administered by constant icv infusion into guinea pig brains by ALZET minipumps for 7 days or 30 days Brain extracts were prepared and total Aβ (Ab40 and Ab42) were measured by ELISA assays
   (A) Loxistatin reduces brain Ab levels. E64d treatment for 7 or 30 days reduced the total Aβ by 31 % and 55% relative to control, respectively (diagonal striped bars)
      Results are shown as mean ± SEM, with * indicating significance at p < 0 05 (by student's t-test)
   (B) CA074Me reduces brain Ab levels. *In vivo* CA074Me treatment for 7 or 30 days reduced total Aβ by 57% and 62% relative to control, respectively (solid bars). After 7 days of treatment, CA074Me significantly reduced total Aβ Data are shown as mean ± SEM, with statistical significance of *p<0 05 (by student's t-test).

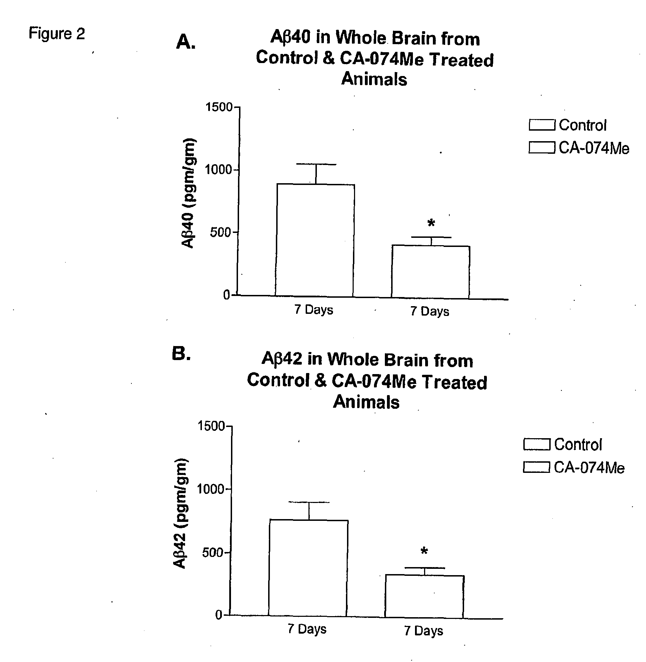

**Figure 2****. CA074Me reduces equally brain Aβ40 and Aβ42 *in vivo.***
   Aβ40 and Aβ42 were individually measured in brain extracts from animals treated with CA074Me or saline control for 7 days. Results are expressed as the mean ± SEM, with statistical significance of * p < 0.05 by the student's t-test
   (A) Reduction of Ab40 by CA074Me. CA074Me treatment (solid bars) significantly decreased Aβ40 in brain extracts, with a 47% reduction.
   (B) Reduction of Ab42 by CA074Me. CA074Me treatment (solid bars) significantly reduced Aβ42 in brain by 56%. Importantly, the mean Aβ40 and Aβ42 levels in control samples did not significantly differ from each other, nor did the mean Aβ40 and Aβ42 levels from the CA074Me-treated animals These data show that the CA074Me treatment reduced each Aβ40 and Aβ42 peptide form to a similar extent.
**Figure 3****. E64d and CA074Me reduces synaptosome Aβ** ***in vivo.*** Synaptosome fractions were purified from brains after treatment with e64d or CA074Me, for 7 or 30 days. Total Aβ levels in synaptosomes were measured by ELISA assays. Results are expressed as the mean ± SEM, with *p < 0.05 for statistical significance (student's t-test).
**Figure 4****. CA074Me reduces brain CTFβ *in vivo.***
   CTFβ was analyzed in brain extracts from animals treated with CA074Me or control for 7 days by western blots..
   (A) CTFb analyses by Western blots Brain extracts from animals treated with CA074Me were subjected to western blots using antibody MAB 1560 The 12 kDa band (indicated by arrow) represents CTFb
   (B) Quantitative evaluation of CTFb. Relative levels of CT Fb were assessed by densitometric scanning of western blots from fig. 4A. CA074Me treatment caused a significant 44% reduction in CTFβ Data are shown as the mean ± SEM based on percent of control, statistical significance assessed with *p < 0.05 (by the student's t test)

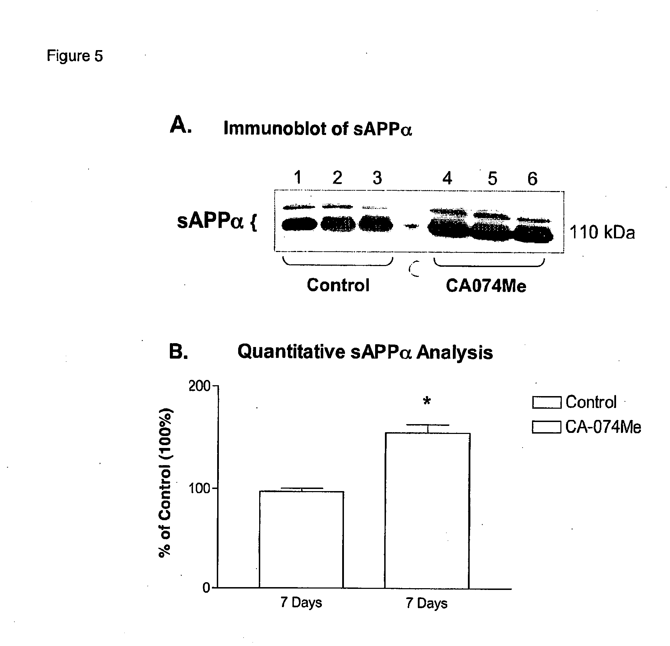

**Figure 5****. CA074Me increases brain sAPPα *in vivo.***
   Levels of sAPPα were analyzed in brain extracts from animals treated with CA074Me and saline control for 7 days.
   (A) Analyses of sAPPa by western blots. Brain extracts were subjected to western blots using antibody directed to the NH₂ terminal domain of APP, antibody MAB 343. The 90 kDa band represents sAPPa.
   (B) Quantitative evaluation of sAPPa Relative levels of sAPPa were determined by densitometric scanning of western blots from fig. 5A CA074Me treatment resulted in a significant increase in sAPPα of 60% above control. Results are expressed as the mean ± SEM based on percent of control, with statistical significance indicated by *p < 0 05 level (by the student's t-test)..

### DETAILED DESCRIPTION OF THE INVENTION

### CDS Prodrug

A prodrug is a pharmacological substance, which is administered in an inactive or less active form Once administered, the prodrug is metabolized in the body *in vivo* to release a biologically active compound (drug). In the context of this invention, a prodrug is also one that can be metabolized by cells or *in vitro* to release a drug. A prodrug has improved absorption, distribution, metabolism or excretion properties (ADME) relative to the drug. The prodrug of this invention has improved ADME for delivery to the brain and central nervous system

A prodrug of the invention contains a moiety that reduces the enzymatic activity of any cysteine protease after being metabolically released from the prodrug.. As such, a prodrug of the invention contains a cysteine protease inhibitor moiety that is active upon release from the prodrug In one embodiment, the prodrug contains a moiety that inhibits enzymatic activity of some, most or all cysteine proteases upon release. The invention also includes a prodrug, wherein the moiety inhibits some, most or all cathepsin proteases upon liberation. In another preferred form, the moiety selectively inhibits cathepsin B after metabolism. Cysteine protease inhibitor moieties of the invention are detailed below.

A prodrug of the invention is a compound which, when metabolized, releases a cysteine protease inhibitor moiety, said compound having the structure: 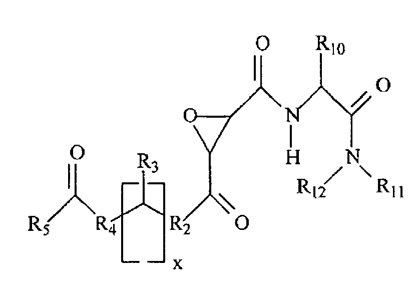 where R₂ is an O, NH, or N substituted with a C₁₋₆ alkyl group, optionally containing one or more aryl groups or heteroatoms;
X is 0, 1 or 2;
R₃, is absent when X is 0 and, when X is 1 or 2, R₃ is H, a C₁₋₆ alkyl group, optionally containing one or more aryl groups or heteroatoms, or an aryl group;
R₄ is absent when X is 0, or, when X is 1 or 2, R₄ is O, NH, or N substituted with a C₁₋₆ alkyl group, optionally containing one or more aryl groups or heteroatoms, or an aryl group; and
R₅ is a dihydropyridine ring attached via a dihydropyridine ring carbon, where:
the dihydropyridine ring carbons are each optionally substituted by a C₁₋₆ alkyl group optionally containing one or more aryl groups or heteroatoms, or an aryl group, or,
taken together, form a 5, 6, or 7 member aromatic or acyclic fused ring optionally containing one or more heteroatoms; and
the dihydropyridine ring nitrogen is substituted by a C₁₋₆ alkyl group, which optionally contains one or more aryl groups or heteroatoms, or an aryl group; and R₁₀ is selected from a C₁₋₆ alkyl group, optionally containing one or more aryl groups or heteroatoms, an aryl group or a phenol group, or forms a part of a leucine or isoleucine group in the compound,
R₁₁ is selected from a C₁₋₁₀ alkyl group, optionally containing one or more aryl groups, heteroatoms, a guadinium group, an imidizol amine group or a propionylamino-acid methyl ester group, and
R₁₂ is H

A prodrug of the invention may also be a compound which, when metabolized, releases a cysteine protease inhibitor moiety, said compound having the structure: 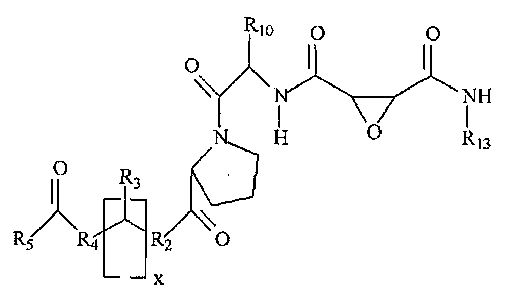 where: R₂ is O, NH, or N substituted with a C₁₋₆ alkyl group, optionally containing one or more aryl groups or heteroatoms;
X is 0, 1 or 2;
R₃, is absent when X is 0, or, when X is 1 or 2, R₃ is H, a C₁₋₆ alkyl group, optionally containing one or more aryl groups or heteroatoms, or an aryl group;
R₄ is absent when X is 0, or, when X is 1 or 2, R₄ is O, NH, or N substituted with a C₁₋₆ alkyl group, optionally containing one or more aryl groups or heteroatoms, or an aryl group; and
R₅ is a dihydropyridine ring attached via a dihydropyridine ring carbon, where:
the dihydropyridine ring carbons are each optionally substituted by a C₁₋₆ alkyl group optionally containing one or more aryl groups or heteroatoms, or an aryl group, or,
taken together, form a 5, 6, or 7 member aromatic or acyclic fused ring optionally containing one or more heteroatoms; and
the dihydropyridine ring nitrogen is substituted by a C₁₋₆ alkyl group, which optionally contains one or more aryl groups or heteroatoms, or an aryl group; and R₁₃ is a C₁₋₆ alkyl group, optionally containing one or more aryl groups or heteroatoms, or an aryl group.
The present invention also relates to a pharmaceutical composition comprising the compounds described above and a pharmaceutically acceptable carrier and a product including the compounds or pharmaceutical compositions described above, for use in treatment of dementia.

The prodrug is metabolized to produce a biologically active moiety having the following structure: 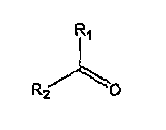 Thus, metabolism of the prodrug results in the release of the cysteine protease inhibitor moiety, R₁, containing the carbonyl R₂ group. As such, a CDS prodrug contains any cysteine protease inhibitor moiety such that, upon release from the prodrug, a functional cysteine protease containing the carbonyl-R₂ group is liberated Thus, a preferred R₂ is O or NH as these form the carboxylic acid or amide moieties, respectively, found on many cysteine protease inhibitors (see Example 1). But functional cysteine protease inhibitors also have at the R₂ position a substituted N. Thus, R₂ also includes a N having a substituent that is any group known in the art to result in a cysteine protease inhibitor. A R₂ also includes a N substituted with a 1-6 alkyl, optionally substituted with one or more aryl, heteroaryl or heteroatoms, which can be optionally substituted with a 1-6 alkyl, optionally containing one or more aryl, heteroaryl, or heteroatoms

Prodrugs generally and their metabolism specifically is well known in the art (see for example, Testa, B., and J.M Mayer, "Hydrolysis in Drug and Prodrug Metabolism: Chemistry, Biochemistry and Enzymology", Wiley Pub., 2003, ISBN 3-90639-025-X). Hydrolysis is the means of metabolism by which the cysteine protease inhibitor is released from the prodrug.

The prodrug of the invention selectively targets the cysteine protease inhibitor to the brain or central nervous system. Using a chemical delivery system (CDS) coupled to the cysteine protease inhibitor, the ratio of brain to periphery drug levels can be increased This offers significant advantages in the treatment of dementia because the drug is concentrated in the brain thereby reducing the dose and toxicity (see for example, Bodor and Buchwald, Barriers to remember: brain-targeting chemical delivery systems and Alzheimer's disease, Drug Discov Today, 7(14):766-74, 2002).

The prodrug of the invention is a CDS prodrug, which exploits the bidirectional properties of the blood brain barrier to lock-in the CDS prodrug in the brain and allow sustained release of the cysteine protease inhibitor. CDS moiety as broadly described above and includes 1,4-dihydrotrigoneline esters and amides linked to cysteine protease inhibitor moieties (see for example, Bodor, Farag, Barros, Wu and Buchwald, In vitro and in vivo evaluations of dihydroquinoline- and dihydroisoquinoline-based targetor moieties for brain-specific chemical delivery systems, J Drug Target, 10(1):63-71, 2002). The 1,4 dihydrotrigoneline esters and amides are hydrophobic and allow me CDS prodrug to readily cross the blood-brain barrier. But the hydrophobic 1,4 dihydrotrigoneline is converted *in vivo* to a hydrophilic quaternary form. This conversion takes place throughout the body via oxidation but without generating active or reactive radical intermediates. In the periphery the charged quaternary form is rapidly eliminated from the body but in the brain the charged form is locked behind the blood-brain barrier. This results in the brain being the only site containing the CDS prodrug and drug after a relatively short period of time. There, the CDS prodrug continues to be metabolized and release the cysteine protease inhibitor As a result, there is a sustained release of the drug in the brain. Thus, the CDS prodrug not only delivers the cysteine protease inhibitor moiety to the brain, it also provides preferential delivery there, thus targeting the brain (Buchwald and Bodor, Physicochemical aspects of the enzymatic hydrolysis of carboxylic esters, Pharmazie, 57(2):87-93, 2002). CDS prodrugs have been applied to deliver antibiotics to the brain (Pop, Wu, Shek and Bodor, Brain-specific chemical delivery systems for beta-lactam antibiotics. Synthesis and properties of some dihydropyridine and dihydroiosquinoline derivatives of benzylpenicillin, J Med Chem, 32(8):1774-81, 1989) (Wu, Pop, Shek and Bodor, Brain-specific chemical delivery systems for beta-lactam antibiotics. In vitro and in vivo studies of some dihydropyridine and dihydroisoquinoline derivatives of benzylpenicillin in rats, J Med Chem, 32(8):1782-8, 1989)

Broadly, the CDS prodrugs of the invention include those in which structure (I) is attached to any of the dihydropryidine ring C of R₅, In select CDS prodrugs, structure (I) is attached to any unsaturated dihydropyridine ring C of R₅.

In another preferred form, the CDS prodrugs include those having this structure: 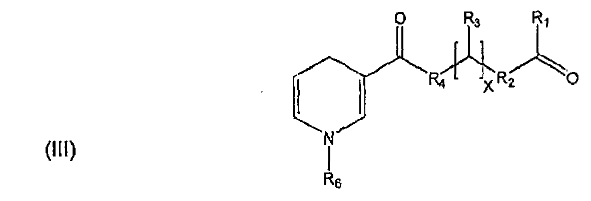 where the variables are as defined above

Another form of CDS prodrug has the following structure: 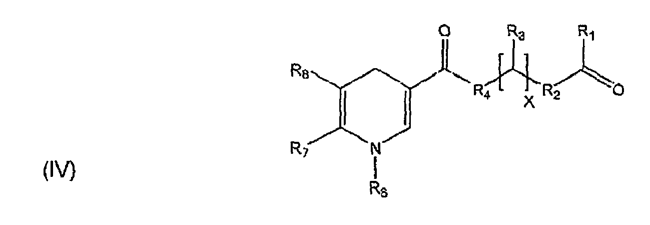 where:
R₇ and R₈ together form the 5, 6, or 7 member aromatic or acyclic fused ring containing one or more heteroatoms and the other variables are as defined above

A CDS prodrug also has the following structure: 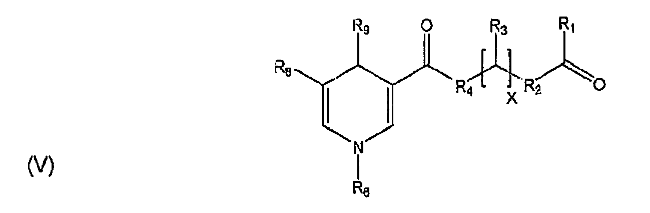 where:
R₈ and R₉ together form the 5, 6, or 7 member aromatic or acyclic fused ring containing one or more heteroatoms and the other variables are as defined above..

A R₁ cysteine protease inhibitor moiety is any compound that contains the carbonyl-R₂ group described above that, upon liberation from the prodrug, is capable of inhibiting a cysteine protease, cathepsin protease or cathepsin B. Large proteinacious cysteine protease inhibitors are known (see, for example, Rzychon, Chmiel and Stec-Niemczyk, Modes of inhibition of cysteine proteases, Acta Biochim Pol, 51(4):861-73, 2004) but generally not suitable as drugs.. Rather, small molecule protease inhibitors generally, and cysteine protease inhibitors specifically are useful as therapeutics (see for example, Abbenante and Fairlie, Protease inhibitors in the clinic, Med Chem, 1(1):71-104, 2005) (Turk Targeting proteases: successes, failures and future prospects, Nat Rev Drug Discov, 5(9):785-99, 2006) (Turk and Guncar, Lysosomal cysteine proteases (cathepsins): promising drug targets, Acta Crystallogr D Biol Crystallogr, 59(Pt 2):203-13, 2003). Thus, preferred cysteine protease inhibitor of the invention is a small molecule suitable for drug development.

Many small molecule cysteine protease inhibitors are known in the art and are preferred for use as the cysteine protease inhibitor moiety in the CDS prodrug (see, for example, Frlan and Gobec, Inhibitors of cathepsin B, Curr Med Chem, 13(19):2309-27, 2006). Among such known small molecule cysteine protease inhibitors, the epoxysuccinyl peptides are preferred. These inhibitors are the most extensively developed small molecule cysteine protease and cathepsin B specific inhibitors. E64, [2*S*,3*S*)-3-(*N*-{(*S*)-1-[*N*(4guanidinobutyl)carbamoyl]3methylbutyl}carbamoyl) oxirane-2-carboxylic acid], was originally isolated from *Aspergillus japonicus* Derivatives of e64 have been described, (for example, in U.S. Patents 6,689,785, 6,387,908, 6,110,967, 5,843,992, 5,679,708, 5,556,853, 4,418,075, and 4,333,879 and Frlan, ibid.).

Thus, a preferred R₁ has the following structure: 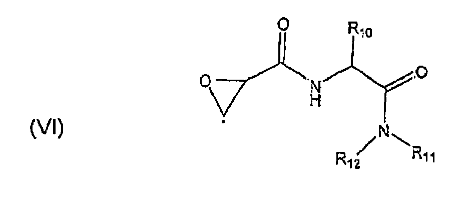 where:
the "•" in structure VI represents the attachment site of R₁ to the prodrug

R₁₀ in preferred prodrug is also a 1·6 alkyl, optionally containing one or more aryl, or heteroatoms, or an aryl. In another preferred prodrug, R₁₀ is a 1-6 alkyl, optionally containing a benzyl or phenol. In a preferred prodrug, R₁₀ is a butyl group. Another preferred R₁₀ forms in structure VI a leucine or isoleucine group. R₁₁ is a 1-10 alkyl, optionally containing one or more aryl, or heteroatoms in a selected prodrug. In another preferred prodrug, R₁₁ is 1-6 alkyl, optionally containing guadinium, imidazol amine group, or a propionylamino-acid methyl ester group. R₁₁ is a pentane group, preferably an isopentane group, in a preferred prodrug. In a preferred prodrug, R₁₂ is H. The SS conformer (trans) of the epoxide of structure VI is preferred

A preferred prodrug is where the cysteine protease inhibitor moiety is e64c (3-[3-methyl-1 -(3-methyl-butylcarbamoyl)-butylcarbamoyl]-oxirane-2-carboxylic acid) with R₂ the O, coupled to structures I through V. A preferred CDS prodrug is where R₁ is the e64c moiety, R₂ the O, coupled to structure III, where R₆ is a methyl, A preferred CDS prodrug is where R₁ is the e64c moiety, R₂ the O, coupled to structure IV, where R₆ is a methyl and R₇ and R₈ together form a fused benzyl or pyridyl ring. A preferred CDS prodrug is where R₁ is the e64c moiety, R₂ the O, coupled to structure V, where R₆ is a methyl and R₈ and R₉ together form a fused benzyl or pyridyl ring..

A CDS preferred prodrug is where R₁ is the e64c moiety, R₂ the O, coupled to structure III, where X is 0, and R₆ is a methyl. A preferred CDS prodrug also is a 1-Methyl-1,4-dihydro-pyridine-3-carboxylic acid 3-[3-methyl-1-(3-methyl-butylcarbamoyl)-butylcarbamoyl]-oxiranecarbonyloxymethy) ester, 1-methyl-1,4-dihydro-pyridine-3-carboxylic acid 1-{3-[3-methyl-1-(3-methyl-butylcarbamoyl)-butylcarbamoyl]-oxiranecarbonyloxy}-ethyl ester, and 3-[3-methyl-1-(3-methyl-butylcarbamoyl)-butylcarbamoyl]-oxirane-2-carboxylic acid 2-[(1-methyl-1,4-dihydro-pyridine-3-carbonyl)-amino]-ethyl ester.

A preferred CDS prodrug is where R₁ is the e64c moiety, R₂ the O, coupled to structure IV, where X is 0, R₆ is a methyl, and R₇ and R₈ together form a fused benzyl ring. A preferred CDS prodrug is also 3-[3-methyl-1-(3-methyl-butylcarbamoyl)-butylcarbamoyl]-oxirane-2-carboxylic acid 2-[(1-methyl-1,4-dihydro-quinoline-3-carbonyl)-amino] ethyl ester, 1-ethyl·1,4-dihydro-[1,7]naphthyridine-3-carboxylic acid ({3-[3-methyl-1-(3-methyl-butylcarbamoyl)-butylcarbamoyl]-oxiranecarbonyl}-amino)-methyl ester, and 1-methyl 1,4-dihydro-quinoline-3-carboxylic acid {3-[3-methyl-1-(3-methyl-butylcarbamoyl)-butylcarbamoyl]-oxiranecarbonyl}-amide

A preferred CDS prodrug is where R₁ is the e64c moiety, R₂ the O, coupled to structure V, where X is 0, R₆ is a methyl, and R₈ and R₉ together form a fused benzyl ring. A preferred CDS prodrug is also 3-[3-Methyl-1-(3-methyl-butylcarbamoyl)-butylcarbamoyl]-oxirane-2-carboxylic acid 2-[(2-methyl-1,2-dihydro-isoquinoline-4-carbonyl)-amino]-ethyl ester

A derivative of e64, CA074, also referred to as CA-074 (1-{3-methyl-2-[(3-propylcarbamoyl-oxiranecarbonyl)-amino]-pentanoyl}-pyrrolidine-2-carboxylic acid), has been developed having increased specificity for inhibiting cathepsin B. As such, prodrugs of CA074 derivatives are a preferred embodiment. CA074 and its derivatives have been described (see for example, in U.S. Patent 5,281,717, Yamamoto, Tomoo, Matsugi, Hara, In, Murata, Kitamura and Ishida, Structural basis for development of cathepsin B-specific noncovalent-type inhibitor: crystal structure of cathepsin B-E64c complex, Biochim Biophys Acta, 1597(2):244-51, 2002, and Frlan, ibid)

Thugs, another preferred R₁ has the following structure: 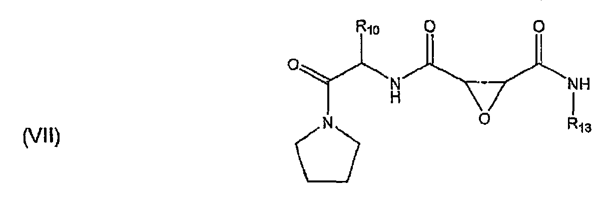 where:
R₁₀ is as defined above, and the "•" in structure VII represents the attachment site of R₁ to the prodrug.

R₁₃ in preferred prodrug is also a 1-6 alkyl, optionally containing one or more aryl, or heteroatoms, or an aryl. In a preferred prodrug, R₁₃ is propyl group, preferably an n-propyl group. The SS conformer (trans) of the epoxide of structure VII is preferred

A preferred prodrug is where the cysteine protease inhibitor moiety is CA074 with R₂ the O, coupled to structures I through V. A preferred CDS prodrug is where R₁ is the CA074 moiety, R₂ the O, coupled to structure III, where R₆ is a methyl, A preferred CDS prodrug is where R₁ is the CA074 moiety, R₂ the O, coupled to structure IV, where R₆ is a methyl and R₇ and R₈ together form a fused benzyl or pyridyl ring. A preferred CDS prodrug is where R₁ is the CA074 moiety, R₂ the O, coupled to structure V, where R₆ is a methyl and R₈ and R₉ together form a fused benzyl or pyridyl ring..

A CDS preferred prodrug is where R₁ is the CA074 moiety, R₂ the O, coupled to structure III, where X is 0, and R₆ is a methyl. A preferred CDS prodrug also is a 1-{3-methyl-2-[(3-propylcarbamoyl-oxiranecarbonyl)-amino]-pentanoyl}-pyrrolidine-2-carboxylic acid 1-[(1-methyl-1,6-dihydro-pyridine-3-carbonyl)-amino]-ethyl ester, 1-{3-methyl-2-[(3-propylcarbamoyl-oxiranecarbonyl)-amino]-pentanoyl}-pyrrolidine-2-carboxylic acid 2-[(1-methyl-1,4-dihydro-pyridine-3-carbonyl)-amino]-ethyl ester, and 1-methyl-1,4-dihydro-pyridine-3-carboxylic acid 1-{3-methyl-2-[(3-propylcarbamoyl-oxiranecarbonyl)-amino]pentanoyl}-pyrrolidine-2-carbonyloxymethyl ester

A preferred CDS prodrug is where R₁ is the CA074 moiety, R₂ the O, coupled to structure IV, where X is 0, R₆ is a methyl, and R₇ and R₈ together form a fused benzyl ring. A preferred CDS prodrug is also oxirane-2,3-dicarboxylic acid 2-({1-[2-(1-isopropyl-1,4-dihydro-quinoline-3-carbonyl)-aminocarbonyl]-pyrrolidine-1-carbonyl}-2-methyl-butyl)-amide) 3-propylamide, 1-{3-Methyl-2-[(3-propylcarbamoyl· oxiranecarbonyl)-amino]-pentanoyl}-pyrrolidine-2-carboxylic acid 2-[(1-methyl-1,4-dihydro-quinoline-3-carbonyl)-amino]-ethyl ester, 1-Propyl-1,4-dihydro-[1,7]naphthyridine-3-carboxylic acid [(1-{3-methyl-2-[(3-propylcarbamoyl-oxiranecarbonyl)-amino]-pentanoyl}-pyrrolidine-2-carbonyl)-amino]-methyl ester.

A preferred CDS prodrug is where R₁ is the CA074 moiety, R₂ the O, coupled to structure V, where X is 0, R₆ is a methyl, and R₈ and R₉ together form a fused benzyl ring. A preferred CDS prodrug is also 1-propyl-1,4-dihydro-[1,7]naphthyridine-3-carboxylic acid [(1·{3·methyl-2-[(3-propylcarbamoyl-oxiranecarbonyl)-amino]-pentanoyl}-pyrrolidine-2-carbonyl)-amino]-methyl ester.

Another preferred CDS prodrug of the invention contains a cysteine protease inhibitor moiety that is an azirdine (see for example, Martichonok, Plouffe, Storer, Menard and Jones, Aziridine analogs of [[trans-(epoxysuccinyl)-L-leucyl]amino]-4-guanidinobutane (E-64) as inhibitors of cysteine proteases, J Med Chem, 38(16):3078-85, 1995, Schirmeister, New peptidic cysteine protease inhibitors derived from the electrophilic alpha-amino acid aziridine-2,3-dicarboxylic acid, J Med Chem, 42(4):560-72, 1999, and Schirmeister and Peric, Aziridinyl peptides as inhibitors of cysteine proteases: effect of a free carboxylic acid function on inhibition, Bioorg Med Chem, 8(6):1281·91, 2000) These compounds have been described as cathepsin protease and cathepsin B inhibitors.

Thus, a preferred R₁ has the following structure: 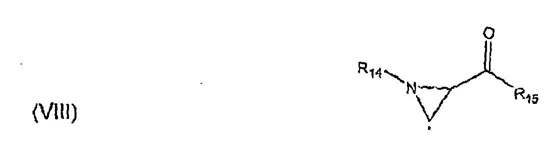 where:
the "•" in structure VII represents the attachment site of R₁ to the prodrug.

R₁₄ in a preferred prodrug is H, Boc-phenylalanine or Boc-phenylalaninealanine. R₁₅ in a preferred prodrug is a OH, benzyl ester, isoamyl ester, or methyl ester or leucine-proline, leucine, each optionally containing a benzyl ester, isoamyl ester or methyl ester in a preferred prodrug in which R₁ is structure VIII, R₂ is the O. In a preferred prodrug the SS conformer (trans) or the RR conformer (cis) of the aziridine structure is preferred.

A preferred CDS prodrug has the azridine moiety, structure VIII, with R₂ the O, coupled to structures I through V A preferred CDS prodrug is where R₁ is the VIII moiety, R₂ the O, coupled to structure III, where R₆ is a methyl. A preferred CDS prodrug is where R₁ is the structure VIII moiety, R₂ the O, coupled to structure IV, where R₆ is a methyl and R₇ and R₈ together form a fused benzyl or pyridyl ring. A preferred CDS prodrug is where R₁ is the structure VIII moiety, R₂ the O, coupled to structure V, where R₆ is a methyl and R₈ and R₉ together form a fused benzyl or pyridyl ring. A preferred CDS prodrug is where R₁ is the VIII moiety, R₂ the O, coupled to structure III, where X is 0, R₆ is a methyl, R₁₄ is Boc-phenylalanine, and R₁₅ is leucine-proline A CDS preferred prodrug is where R₁ is the VIII moiety, R₂ the O, coupled to structure IV, where X is O, R₆ is a methyl, R₁₄ is Boc-phenylalanine, and R₁₅ is leucine-proline.A CDS preferred prodrug is where R₁ is the VIII moiety, R₂ the O, coupled to structure V, where X is 0, R₆ is a methyl, R₁₄ is Boc-phenylalanine, and R₁₆ is leucine-proline

Other CDS prodrugs include those containing other natural products that are cysteine protease inhibitors, such as imizaridine, tokaramide A, or leupeptin.

Any assay known in the art can be used to identify new compounds that are cysteine protease inhibitors, cathepsin protease inhibitors or cathepsin B Inhibitors (see for example, Hook, Toneff, Aaron, Yasothornsrikul, Bundey and Reisine, β-Amyloid peptide in regulated secretory vesicles of chromaffin cells: evidence for multiple cysteine proteolytic activities in distinct pathways for β-secretase activity in chromaffin vesicles, Journal of Neurochemistry. 81:237-256, 2002, and US Patents 6,245,884, and 6,627,409) New compounds that reduce activity in such assays can thus be identified as cysteine protease, cathepsin protease, or cathepsin B inhibitors. Moreover, similar assays can be used to determine a compound's affect, or lack thereof, on proteases that are not cysteine proteases in order to determine the specificity of a compound for inhibiting cysteine proteases, cathepsin proteases or cathepsin B. It is understood that such assays would contain the necessary components for enzymatic activity. For example, assays would include reducing conditions because such conditions are known to be required for cysteine protease activity Moreover, in the case of prodrug testing, assays would include the components necessary for liberation of the active inhibitor from the inactive prodrug.

Assays for measuring enzymatic activity commonly employ a substrate that the protease can cleave and detection of a cleavage product produced by that cleavage. Assays include those in which the substrate contains a structure preferred for cleavage by a particular protease or class of proteases. Such substrates are generally known in the art The cleavage product detected after cleavage of the substrate is a measure of the enzymatic activity of the protease. Detection of the cleavage product can be by any method known in the art, such as, for example, detection of a compound that fluoresces after cleavage from the substrate. Alternatively, the cleavage product can be determined by mass spectrometry. The cleavage product can also be detected by radioactive immune methods..

Assays include those in which a protease is present as a purified or recombinant cysteine or cathepsin B protease Cysteine proteases, cathepsin proteases for use in such assays can be identified using various data bases such as, for example, at the web site merops sanger ac uk. Alternatively, assays include those in which other proteases or other biological molecules are present In addition to a cysteine or cathepsin B. In the latter case, the specificity the cleavage can be determined by means known in the art such as, for example, a known preference by the protease for the substrate producing the cleavage product. The inhibition by compounds known to selectively inhibit cysteine proteases, cathepsin proteases, or cathepsin B can also be compared to new compounds to identity appropriate inhibitors. Further, assays in which peptides known to be produced by proteolytic processing by cysteine or cathepsin B activity can be detected and used to screen for compounds that inhibit that activity and thereby select for biologically active moieties of the present invention. Such assays can be cell based assays in which a proteolytic product known to be produced by cysteine protease cleavage is assayed and are well known In the art.

A new compound is considered a cysteine protease inhibitor, cathepsin protease or cathepsin B inhibitor, if the 50% inhibitory concentration (IC₅₀) of the compound is 10 µM or less for cleavage of an appropriate substrate by such a cysteine, cathepsin or cathepsin B protease in an *in vitro* assay containing the purified or recombinant protease A better inhibitor has an IC₅₀ of 100 nM in such an assay. The selectivity of a new compound for inhibiting cysteine proteases can be determined by methods known in the art. For example in inhibitory activity of such new compounds on non-cysteine proteases, serine-, aspartic-, and metallo proteases, can be determined. A preferred new selective cysteine protease inhibitor has an IC₅₀ concentration of more than 10 µM for-cleavage of an appropriate substrate by a non-cysteine protease in an *in vitro* assay containing the purified or recombinant protease But it is understood that cysteine protease inhibitors may inhibit some non-cysteine proteases and still be selective. A new compound that inhibits cysteine proteases is considered selective if does not inhibit the preponderance of non-cysteine proteases. A preferred new selective cysteine protease inhibitor does not inhibit 95% of known non-cysteine proteases. Similarly, a new cathepsin protease inhibitor is selective if it does not inhibit non-cathepsin proteases at the same level Likewise a new cathepsin B inhibitor does not inhibit non-cathepsin B proteases at that level

As used here, the term "alkyl" includes a univalent radical containing only carbon and hydrogen atoms arranged in a chain. The term "alkyl," including "1-6 alkyl", or "1-10 alkyl", includes both straight and branched chained alkyl groups Preferred alkyl groups include 1, 1-2, 1-3, 1-4, 1-5, 1-6 and 1-10 alkyl groups

The term "aryl" includes any functional or substituent derived from a simple aromatic ring. A preferred alkyl is a phenyl group

A term including the phrase "hetero," such as in "heteroaryl" or the phase "substituted heteroatoms' means that the compound includes atoms other just than carbon and hydrogen Preferred heteroatoms include O, N, and S Thus, for example, a preferred heteroaryl would include a pyridine group

The phrase "one or more" includes at least one but not more than the maximum possible based on chemistry. Preferred among the phrase "one or more" is 1, 1-2, 1-3, and 1-4..

The phrase "any group that results in cysteine protease inhibition" includes any substituent which results in a compound that inhibits cysteine protease activity when metabolized from the prodrug. Substituents that result in such inhibitors can be readily determined by any means known the art for doing so, such as by testing a compound containing the substituent for inhibition in an appropriate protease activity assay such as those described herein. Such substitutents are those that result in compounds, which can be synthesized using known methods or obvious variants of those methods to make them.

The phrase "any group known in the art to result a cysteine protease inhibitor" includes a substituent, which is known in the art to result in a compound or a structurally similar compound that inhibits a cysteine protease Such compounds include those cited in references herein. A "structurally similar compound" is one which would be expected to inhibit cysteine proteases based on its structural similarity to compounds known to inhibit cysteine proteases using any means known and especially those in the fields of medicinal chemistry or pharmacology.

The epoxy and aziridine structures described herein include the stereochemistry inherent In them, that results in various conforms of the structures, including configurations of (R,R), (S,R), (R,S), and (S,S). The present invention includes all of these conformations. In addition, some cysteine protease inhibitors contain an asymmetric carbon atom at positions in addition to the epoxy and aziridine structures In those cases, both R and S conforms are included in the invention

Methods of making the CDS prodrugs of the invention are well known in the art (see for example, references cited above in relation to CDS) and various CDS prodrug synthesis is outlined in Schemes I through IV.But it is understood that modifications can be made to this general approach in order to obtain the desired CDS prodrug. Further, the synthesis of CDS prodrug species is provided in Examples 2 and 3.

Methods of making the cysteine protease inhibitor moiety of the prodrug are also well known in the art.. For example, chemical synthesis of many small molecule cysteine protease inhibitors, especially the expoxide and aziridine inhibitors, are known and provided in the references cited above. In addition, the synthesis of e64c and CA074 moieties is described in Examples 2 and 3. Methods of making natural product cysteine protease inhibitors, such as the aziridine mizaridine, and aldehyde inhibitors, tokaramide A, and leupeptin, include their production by an organism that makes such inhibitors and the subsequent purification of the inhibitor Such organisms can be those found in nature or those genetically engineered to enhance production or modify the structure of the natural product. Methods of purifying the natural product inhibitor are very well known in the art.

In Scheme I, the carboxyl group of the cysteine protease inhibitor moiety is reacted with chloromethyl chlorosulfate to produce the chloromethyl ester. Reaction of this ester with the potassium salt of pyridine-3-carboxylic acid provides the pyridine ester which in turn is methylated and reduced with sodium dithionite to provide the CDS prodrug..

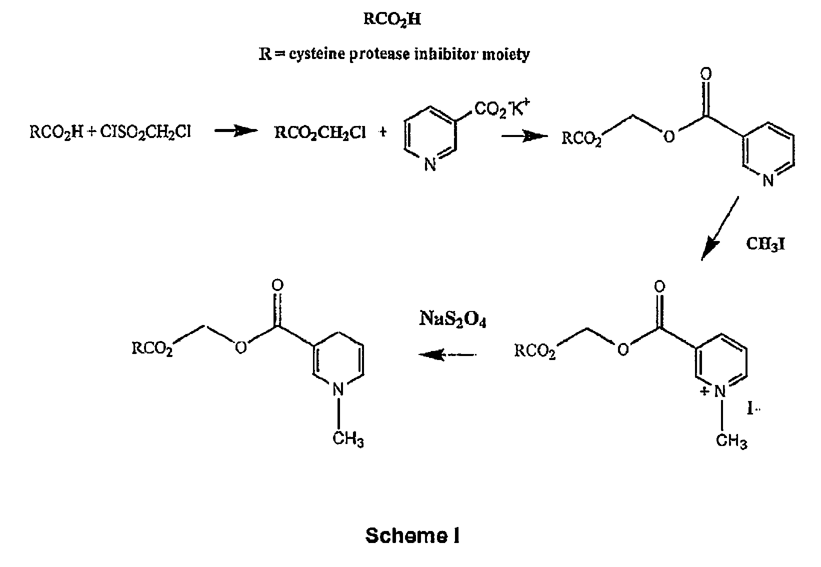

Scheme II depicts the synthesis of an ethyl-bridged CDS prodrug. Reaction of the carboxyl group of the cysteine protease inhibitor moiety with iodoethanol provides the hydroxyethyl ester Reaction with nicotinic anhydride provides the pyridine compound which is processed to the CDS prodrug via alkylation and reduction

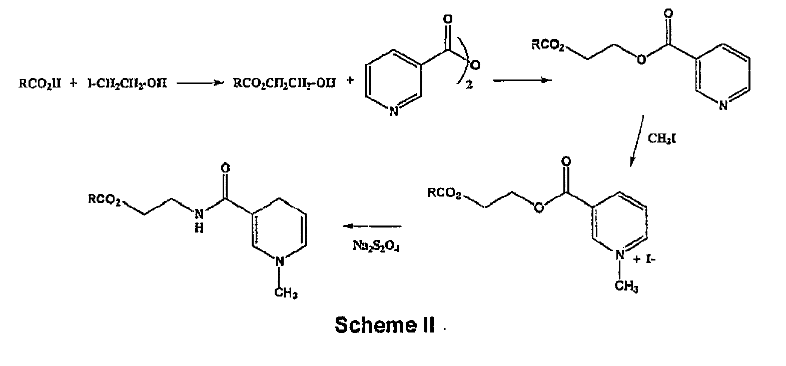

In scheme III, nicotinic amides are prepared by reacting the carboxyl group of the cysteine protease inhibitor moiety with various known (hydroxyalkyl)nicotinamides followed by methylation of the pyridine nitrogen and reduction to the CDS ester

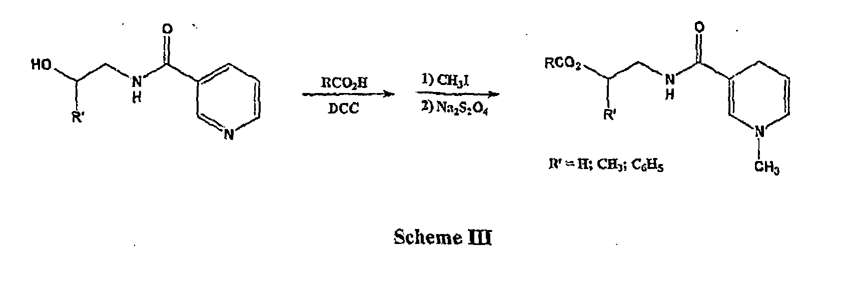

A similar process is followed to prepare the 4-isoquinolinecarboxamides as depicted in scheme IV.

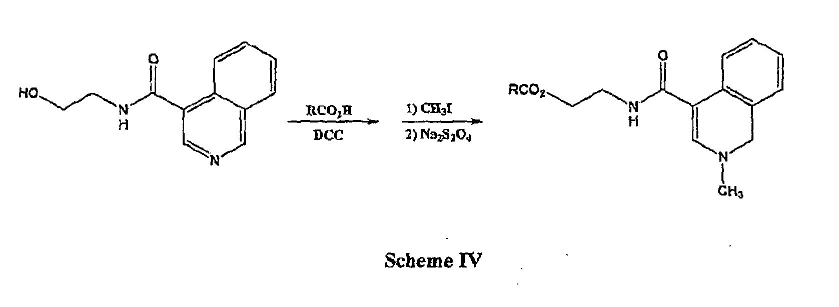

### Pharmaceutical Compositions

The invention is also directed to pharmaceutical compositions containing the prodrugs described above. Pharmaceutical compositions suitable for prodrugs of the invention are generally well known in the art (see, for example, Allen, L.V, Popovich, N G., and Ansel, H., "Ansel's Pharmaceutical Dosages Form and Drug Delivery Systems, Lippincott, William and Wilkins, Pub., 2004, ISBN 0-7817-4612-4, and Gennaro, A., "Remington: The Science and Practice of Pharmacy," 20th ed , Lippincott, Williams and Wilkins, Pub., 2000, ISBN 0·7817-4673-6). Moreover, the special considerations relating to prodrug pharmaceutical compositons are specifically known in the art (see for example, Testa, B, and J.M. Mayer, ibid.).

The prodrug of the invention may be administered orally, topically, parenterally, rectally or by inhalation spray in dosage forms or formulations containing conventional, non-toxic pharmaceutically acceptable carriers, adjuvants and vehicles. The formulation and preparation of any of this broad spectrum of dosage forms into which the subject prodrugs can be disposed is well-known to those skilled in the art of pharmaceutical formulation.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide a pharmaceutically elegant and palatable preparation.

Formulations for oral use include tablets which contain the active ingredient in admixture with non-toxic pharmaceutically acceptable excipients These excipients may be, for example, inert diluents, such as calcium carbonate, sodium chloride, lactose, calcium phosphate or sodium phosphate; granulating and disintegrating agents, for example, potato starch, or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin, or olive oil

Aqueous suspensions usually contain the active materials in admixture with appropriate excipients. Such excipients are suspending agents, for example, sodium carboxymethylcellulose, methylcellulose, hydroxypropylmethylcellulose, sodium alginate, polyvinylpyrrolidone, gum tragacanth and gum acacia; dispersing or wetting agents which may be a naturally-occurring phosphatide, for example, lecithin; a condensation product of an alkylene oxide with a fatty acid, for example, polyoxyethylene stearate; a condensation product of ethylene oxide with a long chain aliphatic alcohol, for example, heptadecaethyleneoxycetanol; a condensation product of ethylene oxide with a partial ester derived from fatty acids and a hexitol such as polyxyethylene sorbitol monooleate; or a condensation product of ethylene oxide with a partial ester derived from fatty acids and hexitol anhydrides, for example, polyoxyethylene sorbitan monooleate. The aqueous suspensions may also contain one or more preservatives, for example, methyl, ethyl or n-propyl p-hydroxybenzoate; one or more colouring agents; one or more flavouring agents; and one or more sweetening agents such as sucrose or saccharin.

Oily suspension may be formulated by suspending the active ingredient in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol Sweetening agents such as those set forth above, and flavouring agents may be added to provide a palatable oral preparation These compositions may be preserved by the addition of an antioxidant such as ascorbic acid

Dispersible powders and granules suitable for preparation of an aqueous suspension by the addition of water provide the active ingredient in admixture with a dispersing or wetting agent, suspending agent and one or more preservatives Suitable dispersing or wetting agents and suspending agents are exemplified by those already mentioned above Additional excipients, for example, sweetening, flavouring and colouring agents, may also be present.

The pharmaceutical compositions of the invention may also be in the form of oil-in water emulsions. The oily phase may be a vegetable oil, for example, olive oil or arachis oils, or a mineral oil, for example, liquid paraffin or mixtures of these. Suitable emulsifying agents may be naturally-occurring gums, for example, gum acacia or gum tragacanth; naturally-occurring phosphatides, for example, soybean licithin; and esters including partial esters derived from fatty acids and hexitol anhydrides, for example, sorbitan mono-oleate, and condensation products of the said partial esters with ethylene oxide, for example, polyoxyethylene sorbitan monooleate. The emulsions may also contain sweetening and flavouring agents.

Syrups and elixirs may be formulated with sweetening agents, for example glycerol, sorbitol or sucrose. Such formulations may also contain a demulcent, a preservative and flavouring and colouring agents, The pharmaceutical compositions may be in the form of a sterile injectable aqueous or oleagenous suspension. This suspension may be formulated according to the known art using those suitable dispersing or-wetting agents and suspending agents which have been mentioned above The sterile injectable preparation may be a sterile injectable solution or suspension in a non-toxic parenterally acceptable diluent or solvent Among the acceptable vehicles and solvents that may be employed are water, 1,3-butanediol, Ringer's solution and isotonic sodium chloride solution. In addition, sterile fixed oils are conventionally employed as a solvent or suspending medium. For this purpose any bland fixed oil may be employed including synthetic monoor diglycerides. Fatty acids such as oleic acid also find use in the preparation of injectibles.

The pharmaceutical compositons may also be administered in the form of suppositories for rectal administration of the drug These compositions can be prepared by mixing the drug with a suitable nonirritating excipient which is solid at ordinary temperatures but liquid at the rectal temperature and will therefore melt in the rectum to release the drug, for example, cocoa butter, or adeps solidus polyethylene glycols..

From the foregoing description, one of ordinary skill in the art can easily ascertain the essential characteristics of the present invention and, without departing from the spirit and scope thereof, can make various changes and/or modifications of the invention to adapt it to various usages and conditions As such, these changes and/or modifications are properly, equitably and intended to be within the full range of equivalence of the following claims.

The amount of active ingredient that may be combined with the carrier materials to produce a single dosage form will vary depending upon the patient treated and the particular mode of administration For example, a formulation intended for the oral administration of humans may contain from 5 mg to 5 gm of the active agent compounded with an appropriate and convenient amount of carrier material, which may vary from about 5 to about 95% of the total composition.. Other dosage forms such as ophthalmic dosage forms contain less active ingredient such as for example from 0.1 mg to 5 mg. Dosage unit forms will generally contain between from about 0.1 mg to about 500 mg of active ingredient.

It will be understood, however, that the specific dose level for any particular patient will depend upon a variety of factors including the activity of the specific compound employed, the age, body weight, general heath, sex, diet, time of administration, route of administration, rate of excretion, drug combination and severity of the dementia

### Treatment of Dementia, Alzheimer's disease (AD) and vascular dementia (VD)

Dementia is the progressive decline in cognitive function due to damage or disease in the brain beyond what might be expected from normal aging Particularly affected areas may be memory, attention, language and problem solving, although particularly in the later stages of the condition, affected persons may be disoriented in time (not knowing what day, week, month or year it is), place (not knowing where they are) and person (not knowing who they are)

The cost of caring for dementia patients is estimated to be $156 billion by 2003 world-wide. That cost is expected to increase substantially as the population of industrialized nations ages, because dementia is a disease of the aged.

Dementia is a non-specific term that encompasses many disease processes with the leading cause of dementia due to Alzheimer's disease (AD) AD is a neurodegenerative disease characterized by progressive cognitive deterioration together with declining activities of daily living and neuropsychiatric symptoms or behavioral changes. The most striking early symptom is short term memory loss (amnesia), which usually manifests as minor forgetfulness that becomes steadily more pronounced with illness progression, with relative preservation of older memories As the disorder progresses, cognitive (intellectual) impairment extends to the domains of language (aphasia), skilled movements (apraxia), recognition (agnosia), and those functions (such as decision-making and planning) closely related to the frontal and temporal lobes of the brain as they become disconnected from the limbic system, reflecting extension of the underlying pathological process.. The AD pathological process consists principally of neuronal loss or atrophy, principally in the temporoparietal cortex, but also in the frontal cortex, together with an inflammatory response to the deposition of amyloid plaques and neurofibrillary tangles.

There is currently no cure for AD, although there are drugs that offer symptomatic benefit, specifically with respect to short-term memory impairment. Unfortunately current drugs produce only temporary effects and do not stop the ultimate progression of the disease. Current FDA approved drugs for AD include, for example, acetylcholinesterase inhibitors and NMDA antagonists.

AD, and the resulting dementia, is thought to be caused by the abnormal accumulation of β-amyloid peptides (Aβ) in the brain. Aβ consist of 3 peptides, Aβ40, Aβ42 and Aβ43, with Aβ40 and Aβ42 the more abundant and important forms. Aβ are a primary component of senile plaques that form in AD brain and which are a hallmark of the disease. Aβ are neurotoxic and result in neuronal cell death and the pathological neuronal structures seen in AD brain, called neurofibrillary tangles, which are also characteristic of the disease Moreover, individuals carrying genetic mutations, which result in increased brain Aβ, develop AD at earlier age than the AD patient population generally As such, reducing brain Aβ are thought to be effective at stopping the progression of AD and may reverse dementia that has already occurred.

Recently, cysteine protease inhibitors, generally, and cathepsin B inhibitors, specifically, have been shown to reduce Aβ production in vitro and by cells secreting Aβ (see, for example, U.S Patent Application Publication 2004/0248232, Hook and Reisine, Cysteine Proteases Are the Major β-Secretase in the Regulated Secretory Pathway That Provides Most of the β-Amyloid in Alzheimer's Disease: Role of BACE 1 In the Constitutive Secretory Pathway, Journal of Neuroscience Research, 74:393-405, 2003, and Hook, Toneff, Bogyo, Medzihradszky, Nevenu, Lane, Hook and Reisine, Inhibition of cathepsin B reduces β-amyloid production in regulated secretory vesicles of neuronal chromaffin cells: evidence for cathepsin B as a candidate β secretase of Alzheimer's disease Biological Chemistry, 386:931-940, 2005). Aβ are proteolytically produced by cleavage of a precursor protein called amyloid precursor protein (APP). Cysteine proteases and cathepsin B, specifically, are thought to be, in part, responsible for that proteolytic processing and thus cysteine protease and cathepsin B inhibitors reduce Aβ by reducing proteolytic production. As such, cysteine protease and cathepsin B inhibitors are potentially effective AD agents In particular, cathepsin B specifically is thought to cut the Aβ amino terminal end and is thus, thought to be a β-secretase, the term used in the art to refer to those proteases that cleave the amino terminal end of Aβ. All three Aβ have the same amino terminal end and differ only at the carboxyl terminal end. Moreover, cysteine proteases and cathepsin B specifically is thought to act as a β-secretase in the neuronal regulated secretory pathway, the pathway by which most Aβ is thought to be produced

But the effectiveness of cysteine protease inhibitors or cathepsin B inhibitors at reducing brain Aβ had not been validated *in vivo* As such, there was some question as to whether cysteine protease inhibitors or cathepsin B inhibitors would be useful for treating AD.. Here, cathepsin inhibitors and cathepsin B inhibitors, specifically, are validated as effective at reducing brain Aβ in vivo and, as such, are effective for treating AD. Using the guinea pig animal model of APP processing, the general cysteine protease inhibitor, e64d (also known as loxistatin, EST, and oxiranecarboxylic acid, 3-[[[(1S)-3-methyl-1-[[(3-methylbutyl)amino]carbonyl]butyl]amino]carbonyl], ethyl ester) and ethyl ester of e64c L-Proline, N[[(2S,3S)3[(propylamino)carbonyl]oxiranyl]carbonyl]-L-isoleucyl ,methyl ester) significantly reduces brain Aβ after direct infusion into the brain (icv) for 7 or 30 days. E64d and CA074Me are the ethyl and methyl esters of e64c and CA074, respectively These esters are metabolically converted to e64c and CA074 upon administration.. The results are shown in Examples 4 through 8

The guinea pig is an excellent model of human APP processing and human Aβ production (see, for example, Beck, Bigl and Rossner, Guinea pigs as a nontransgenic model for APP processing in vitro and in vivo, Neurochem Res, 28(3-4):637-44, 2003) Using that model, cholesterol lowering statins drugs were found to reduce brain Aβ by similar amount shown here for cysteine protease and cathepsin B inhibitors. Based on the results from the guinea pig model, statins have subsequently gone into clinical trials for AD treatment (see for example Fassbender, Simons, Bergmann, Stroick, Lutjohann, Keller, Runz, Kuhl, Bertsch, von Bergmann, Hennerici, Beyreuther and Hartmann, Simvastatin strongly reduces levels of Alzheimer's disease beta amyloid peptides Abeta 42 and Abeta 40 in vitro and in vivo, Proc Natl Acad Sci U S A, 98(10):5856-61, 2001 and www.clinicaltrials.gov, Lipitor, or Simvastatin). As such, the results here reasonably predict that cysteine protease inhibitors and cathepsin B inhibitors are effective AD therapeutics

The second leading cause of dementia is vascular dementia (VD), also known as ischemic vascular dementia or multi-infarct dementia These terms refer to a group of syndromes caused by different mechanisms all resulting in vascular lesions in the brain. The main subtypes of VD described at the moment are: vascular mild cognitive impairment, multi-infarct dementia, vascular dementia due to a strategic single infarct, vascular dementia due to hemorrhagic lesions, small vessel disease (which includes vascular dementia due to lacunar lesions and Binswanger disease), and mixed AD and VD. Vascular lesions can be the result of diffuse cerebrovascular disease or focal lesions (or a combination of both, which is what is observed in the majority of cases). Mixed dementia is diagnosed when patients have evidence of AD and cerebrovascular disease, either clinically or based on neuroimaging evidence of ischemic lesions. In fact VD and AD often coexist, especially in older patients with dementia

For VD, the prevention of further cerebrovascular lesions is the primary therapy. This includes administering antiplatelet drugs and controlling major vascular risk factors (hypertension, hypercholesterolemia, smoking and diabetes mellitus to mention a few). The ischemia that results from VD causes neuronal cell death and resulting dementia. Reducing the neurological damage caused by the ischemia is thought to be effective for treating dementia..

Cysteine protease inhibitors and cathepsin B inhibitors have been shown effective in protecting brain neurons during ischemia. In particular, e64c and CA074 have been shown to protect hippocampus neurons from damage in a primate ischemic animal model (see for example, Tsuchiya, Kohda, Yoshida, Zhao, Ueno, Yamashita, Yoshioka, Kominami and Yamashima, Postictal blockade of ischemic hippocampal neuronal death in primates using selective cathepsin inhibitors, Exp Neurol, 155(2):187-94, 1999, and Yoshida, Yamashima, Zhao, Tsuchiya, Kohda, Tonchev, Matsuda and Kominami, Primate neurons show different vulnerability to transient ischemia and response to cathepsin Inhibition, Acta Neuropathol (Berl), 104(3):267-72, 2002). Thus, these results reasonably predict that cysteine protease inhibitors and cathepsin B inhibitors are effective for treating neuronal cell death due to ischemia and VD

Moreover, cysteine proteases and cathepsin B are useful therapeutic targets because their inhibition is not lethal. For example, e64d, a general cysteine protease inhibitor also known as loxistatin and EST, successfully passed Phase I clinical trials in Japan (see for example Miyahara, Shimojo, Toyohara, Imai, Miyajima, Honda, Kamegai, Ohzeki and Kokatsu, Phase I tudy of EST, a new thiol protease inhibitor - the 2nd report: safety and pharmacokinetics in continuous administration, Rinsho Yakuri, 16(3):537-546, 1985) Those studies were in preparation for its use in treating muscular dystrophy. However, after a Phase III study, further testing was halted due, in part, to a low efficacy for muscular dystrophy (see for example, Scrip, 1765:11, 1992). Cathepsin B inhibitors are also not likely to be lethal, because mice in which cathepsin B has been knocked out appear and behave normally (see for example, Reinheckel, Deussing, Roth and Peters, Towards specific functions of lysosomal cysteine peptidases: phenotypes of mice deficient for cathepsin B or cathepsin L, Biol Chem, 382(5):735-41, 2001).

Nonetheless, there are concerns about the toxicity of cysteine protease inhibitors, especially those such as the epoxide and arizidine compounds that covalently bind to the protease Such covalent binding is thought to make the compound more likely to cause toxic side effects, especially in the liver And indeed, at high dose, liver toxicity was found in rats using e64d (see for example Fukushima, Arai, Kohno, Suwa and Satoh, An epoxysuccinic acid derivative(loxistatin) induced hepatic injury in rats and hamsters, Toxicol Appl Pharmacol, 105(1): 1-12, 1990). Such concerns are magnified in the treatment of AD and VD patients because these patients are elderly and often frail.

The CDS prodrugs of the present invention provide significant advantages in the treatment of these patients because of significantly lower systemic toxicity. As discussed above, the CDS prodrugs of the invention are not reactive In and rapidly clearead from the systemic system, thereby minimizing hepatic exposure. Moreover, CDS prodrugs are targeted to the central nervous system where they provide sustained release of the inhibitors further reducing toxic concerns because of reduced effective doses in the circulation.

The CDS prodrugs of the invention thus, can be used to treat, which includes to ameliorate or prevent, dementia, AD and VD. It is understood that in these conditions and diseases, even temporary reduction in symptoms or a delay in the progression of the disease is significant Moreover, it is also appreciated that these conditions take a long time to develop and thus methods that prevent the condition or disease are also with the scope of the invention..

The effectiveness of the CDS prodrugs in treating these conditions and diseases can be assessed by any means known in field (see for example www.clinicaltrials.gov) For example, cognitive tests have been developed for such, including, for example, the Mattis Dementia Rating Scale and the Mini-Mental State Examination Neuroimaging methods have also been developed for evaluating dementia, AD and VD and can be used such as,magnetic resonance imaging and positron emission tomography Further, biomarkers associated with these conditions and diseases can be monitored by any means known, such as imaging or chemical analysis, to determine the effect of the CDS prodrugs For example, Aβ is a biomarker, which can be monitored. Of course, brain cysteine protease activity and cathepsin B activity can also be monitored to asses effectiveness Moreover, methods such as bioinformatics and proteomics can be used determine other substances that are biomarkers. For example, in AD, proteomic analysis has found that cerebral spinal fluid levels of cathepsin B are elevated in AD (see for example Zhang, Goodlett, Quinn, Peskind, Kaye, Zhou, Pan, Yi, Eng, Wang, Aebersold and Montine, Quantitative proteomics of cerebrospinal fluid from patients with Alzheimer disease, J Alzheimers Dis, 7(2): 125-33; discussion 173-80, 2005). A ventriculoperitonial shunt is useful for obtaining cerebrospinal fluid in order to assess such biomarkers directly.

### EXAMPLE 1

This example demonstrates the release of a cysteine protease inhibitor from the prodrug. In this case, the cysteine protease inhibitor is e64c (3-[3-methyl-1-(3-methyl-butylcarbamoyl)-butylcarbamoyl]·oxirane-2-carboxylic acid). This inhibitor contains a carboxylic acid group at the epoxide ring A prodrug form of e64c as incorporated into structure I has the following structure:

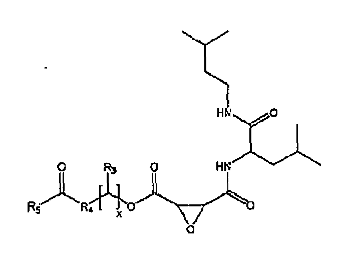

In this case, R₂ is O and, upon metabolism, the carbonyl-R₂ group becomes the carboxylic acid group of e64c and a functional inhibitor having the following structure is released:

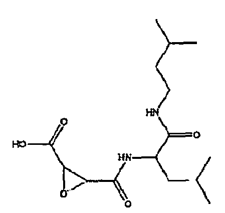

### EXAMPLE 2

This example provides a synthesis of the protease inhibitor e64c and various exemplary CDS prodrugs of e64c.
E64c 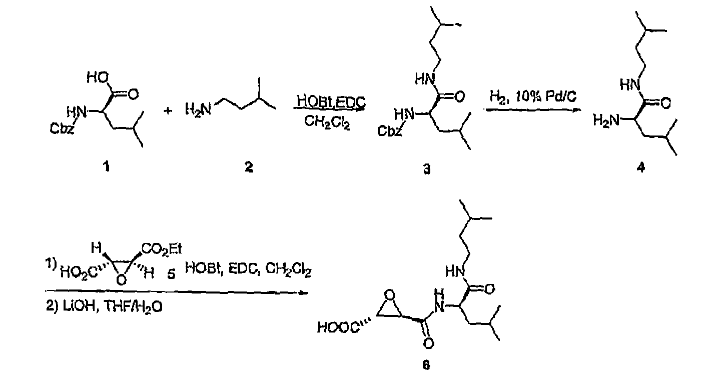 CDS E64c Prodrugs

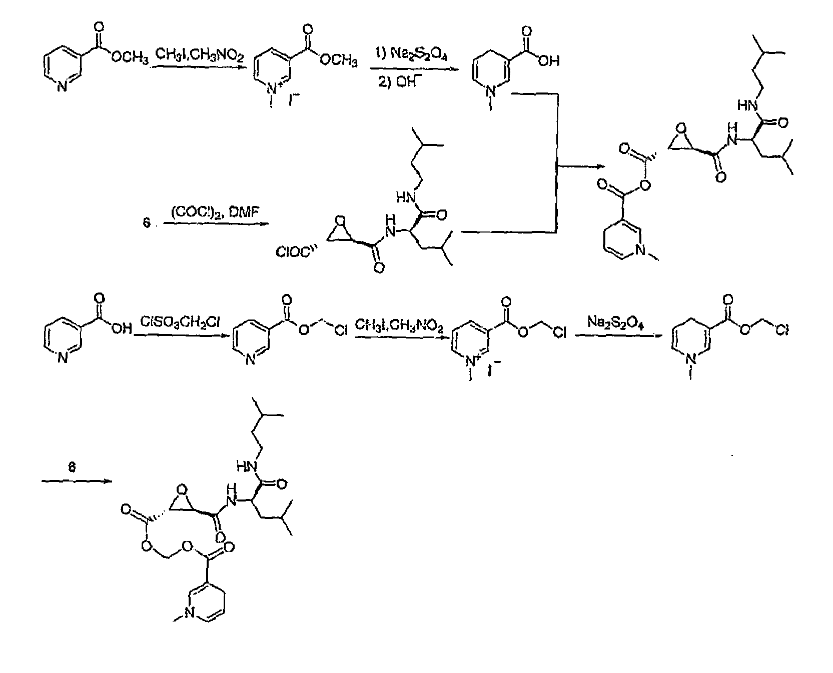

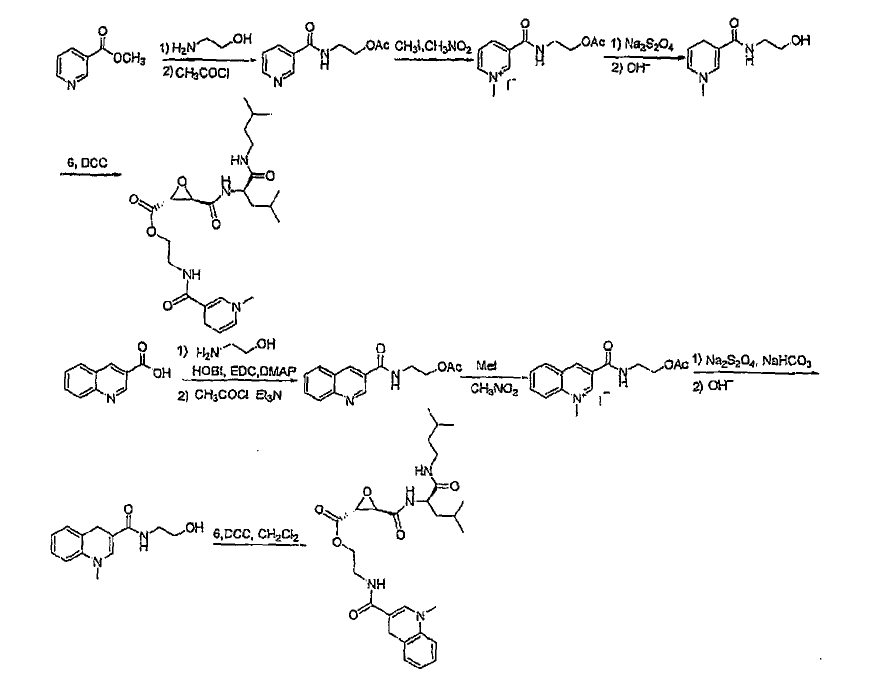

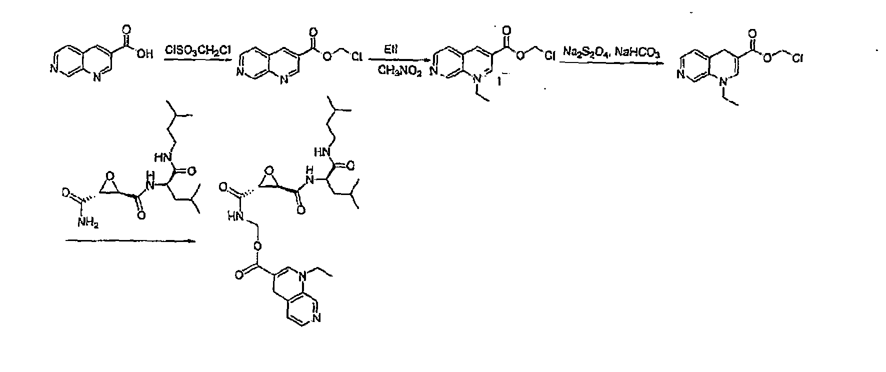

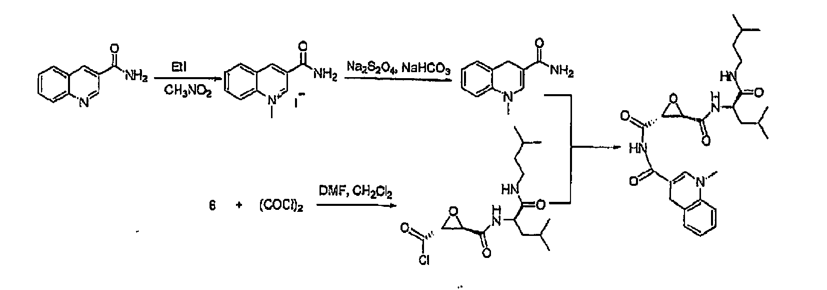

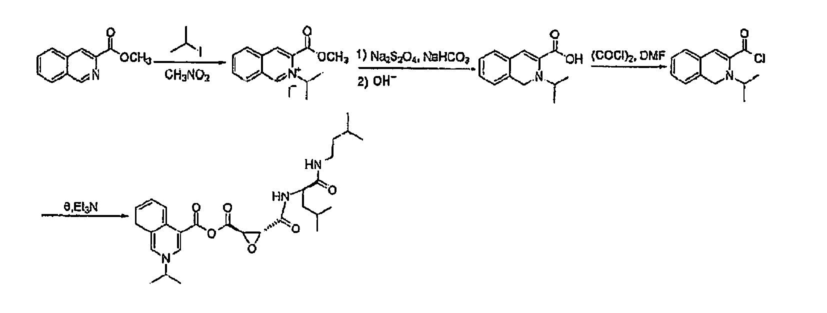

### EXAMPLE 3

This example provides a synthesis of the protease inhibitor CA074 and various exemplary CDS prodrugs of CA074
CA074

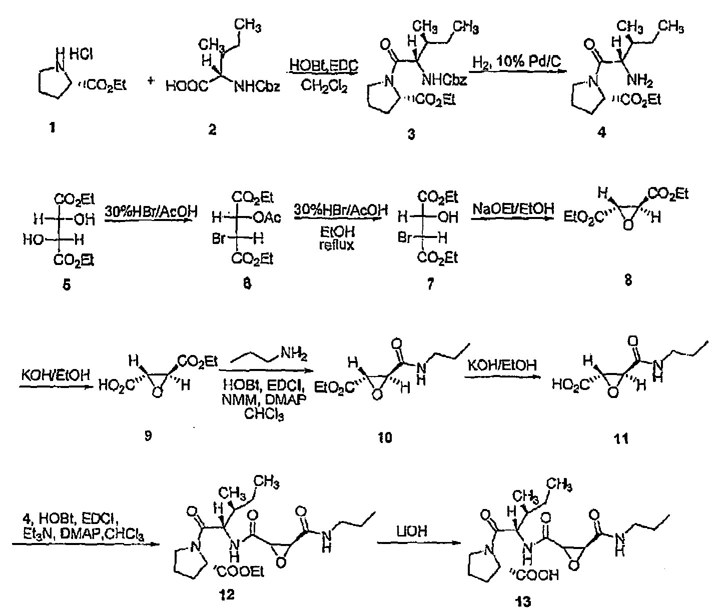

### Synthesis of: dipeptide Cbz-Ile-Pro-Et (3)

To a stirred solution of triethylamine (26 9 mL, 195 mmol) in CH₂Cl₂ (350 mL), ethyl) L-proline 1(18.276g), Cbz·Ile 2 (34.49 g, 130 mmol), DMAP (1.612g, 13.0 mmol), HOBt (26 34g, 195 mmol) and EDC (37.2g, 195 mmol) were added. After stirring overnight, the solvent was removed in vacuo the residue obtained was redissolved in EtOAc and washed with citric acid aq (10%) and then saturated NaHCO₃ and brine. The organic phase was dried over anhydrous MgSO4 and concentrated. Purification by flash chromatography with petroleum ether/EtOAc (8:1, v/v) gave the desired product 3 18 37 g (83%) as yellow oil, ES-MS: 391.2 [M+H⁺]

### Ile-Pro-Et (4)

The dipeptide products 3 obtained above (13 0 g, 33.3 mmol) was hydrogenated in MeOH (150 mL) over 10% Pd/C (13 g). The catalyst was filtered off though a celite column and the solvent was removed in vacuo to provide compound 4, ES-MS: 257.2 [M+H⁺], which was used directly and rapidly in the next step without further purification

### Diethyl 2-acetoxy-3-bromosuccinate(6)

30% HBr/AcOH (440.5 mL) was added dropwise with stirring to D-(-)-tartrate 5 (150 g, 727.87 mmol) in an ice bath. Then the mixture was stirred overnight at room temperature. Ice water 700 mL was added to the mixture carefully. The aqueous phase was extracted with ether (8 X 300 mL) The combined ether layer was washed with water (2 X 200 mL) and brine (2 X 200 mL), dried over anhydrous MgSO4, concentrated in vacuo to provide compound 6 as an oil (194g , 94%), ES-MS: 311 1 [M+H⁺], which was pure enough for the next step.

### Diethyl 2-bromo-3-hydroxysuccinate(7)

To a solution of compound 6 (194 g, 626 mmol) in EtOH (730 mL), 30% HBr/AcOH (71 mL) was added dropwise. Then the reaction mixture was stirred under reflux for 4 h. After concentration in vacuo, the crude oil was distilled under reduced pressure The desire product was collected with bp 125-134°C/4mmHg (133.25g, 79%), ES-MS: 269.0 [M+H⁺]

### (25,3S)-diethyl oxirane-2,3-dicarboxylate(8)

Mental sodium (13.334 g, 579 mmol) was dissolved carefully in an absolute EtOH (332 mL) and the solution was cooled to 0-5 °C in an ice bath A solution of compound 7 (133.25 g, 497 mmol) in EtOH (115 mL) was added dropwise. After stirring at room temperature for 2 h, HOAc (100 mL.) was added. After concentration in vacuo at room temperature, ice water (500 mL) was added and then extracted with ether (6 X 300 mL) The combined organic phase was washed with brine, dried over anhydrous MgSO4, concentrated in vacuo. Distillation under reduced pressure gave the pure product with bp 100-124°C/3mmHg (60 7g, 65%), ES-MS: 189 2 [M+H⁺].

### (2S,3S)-3-(ethoxycarbonyl)oxirane-2-carboxylic acid(9)

KOH (18.061 g, 322 mmol) was dissolved in absolute EtOH (300 mL) With stirring, a solution of compound 8 (60.659 g, 322 mmol) in absolute EtOH (466 mL) was added dropwise over 2 h at 0-5 °C. After continuous stirring at room temperature for 2 h, the reaction mixture was concentrated in vacuo at room temperature and the residue was diluted with ether (2000 mL). The undissolved solid was filtered off and the solvent was evaporated to dryness to give a white solid (53g) 5% KHSO₄ was added to acidify the white solid and the solution was saturated with solid NaCl. The free acid was extracted with EtOAc (6 X 300 mL) and the combined organic phase was washed with water (2 X 300 mL), dried over anhydrous MgSO4. After concentration, the crude product was obtained as an oil (40.69g, 79%), ES-MS: 161.1 [M+H⁺], which was used directly in the next step without further purification.

### (2S,3S)-ethyl 3-(propylcarbamoyl)oxirane-2-carboxylate(10)

Monoethyl (2S, 3S)-trans-epoxysuccinate 9 (10 g, 62 mmol) and n-PrNH₂ (7.387 g, 10.361 mL, 62 mmol) was mixed in CHCl₃ (350 mL) The micture was cooled to 0-5 °C in ice bath. DMAP (0.775 g, 6 mmol), HOBt (8 435 g, 62 mmol), NMM (6.953 g, 7.616 mL, 69 mmol) and EDC.HCl (13.176 g, 69 mmol) were added. After stirring overnight, the reaction mixture was concentrated in vacuo. The residue was redissolved in EtOAc (600 mL) and washed with 5% KHSO₄ aq., saturated NaHCO₃ (2 X 100 mL) and brine (2 X 100 mL), dried over anhydrous MgSO4. Concentration and purification by recrystallization from petroleum ether/EtOAc afforded the desired compound 11 (10.6g, 35%) as white soild, Mp 84-85°C. ES-MS: 202.1 [M+ H⁺]; ¹H NMR (CDCl₃)δ: 6.10 (brs, 1 H), 4.26 (qd, J = 5, 1.8 Hz, 2 H), 3.68 (d, J = 2 1 Hz, 1 H), 3 47 (d, J = 2..M Hz, 1 H), 3 23 (q, J = 6.9 Hz, 2 H), 1.59-1.47 (m, 2 H), 1.31 (t, J = 7.5 Hz, 3H), 0 92 (t, J = 7.5 Hz, 3H).

### (2S,3S)-3-(propylcarbamoy)oxirane-2-carboxylic acid (11)

A solution of KOH (3 028 g, 54 mmol) in EtOH (250 mL) was added dropwise to the solution of compound 10 (5,437 g, 27 mmol) in EtOH (185 mL) at 0-5 °C with stirring After completion, the solution was warmed to room temperature and stir was continued for 1 h The solvent was evaporated in vacuo at room temperature and diluted with water (300 mL). The solution was acidified with 5% KHSO₄ and saturated with NaCl, then extracted with EtOAc (5 X 120 mL). The combined organic phase was washed with brine (2 X 100 mL.), dried over anhydrous MgSO4. After concentration, the desired product was obtained as an oil (4.4g, 95%), ES-MS: 174.0 [M+H⁺], which was used directly in the next step without further purification

### Coupling with EDC/HOBt: Synthesis of compound 12

To a solution of the mono-amidated epoxysuccinate (1.687 g, 19 mmol) and dipeptide 4 (5 g, 19 mmol) in CHCl₃ (200 mL), HOBt (1.318 g, 19 mmol), Et₃N (1.086g, 1.496 mL, 21 mmol) and DMAP (0.121 g, 2 mmol) were added After cooling in an ice bath EDC (2.057 g, 21 mmol) was added and the reaction mixture was stirred overnight at room temperature. The solvent was removed In vacuo and the residue was dissolved in EtOAc (600 mL). The organic phase was washed with 5% KHSO₄ aq., saturated NaHCO₃, brine (2 X 100 mL), and then dried over anhydrous MgSO4. After concentration, the residue was purification by flash chromatography with petroleum ether/EtOAc afforded compound 12 as an oil (3.878 g, 75%), ES-MS: 412 2 [M+H⁺].

### Synthesis of compound 13

To a solution of Compound 12 (2.305 g, 5.6 mmol) in THF (200 mL.) and water (100 mL), LiOH.H₂O (0.471 g, 112 mmol) was added After stirred for 5 h, the reaction solution was acified with 5% KHSO₄ to Ph 2 and then saturated with NaCl The aqueous phase was extracted with EtOAc, washed with water and brine, dried over anhydrous MgS04 and evaporated to afford compound 13 (1 8 g, 90%), ES-MS: 384 1 [M+H⁺].

### CDS CA074 Prodrugs

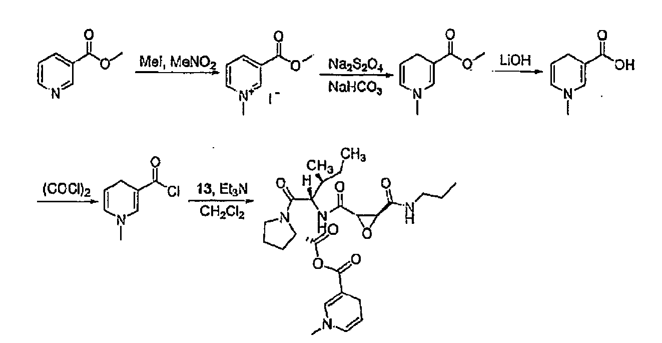

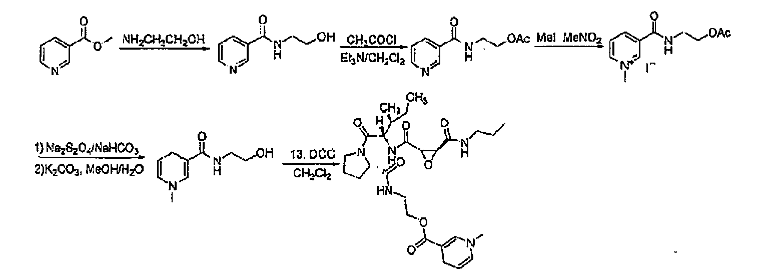

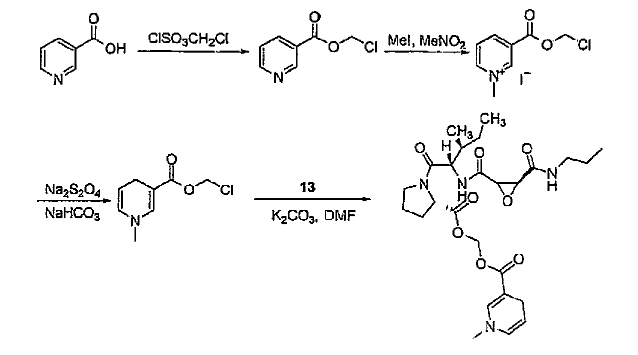

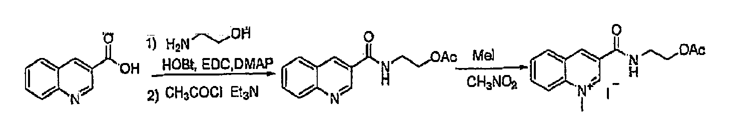

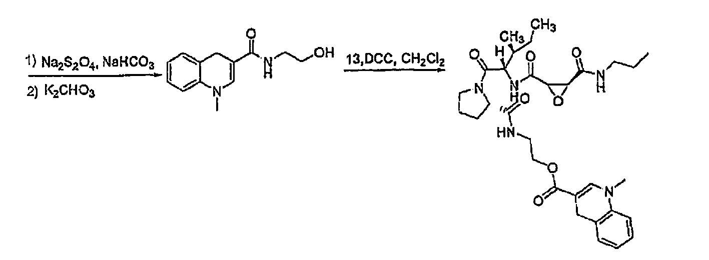

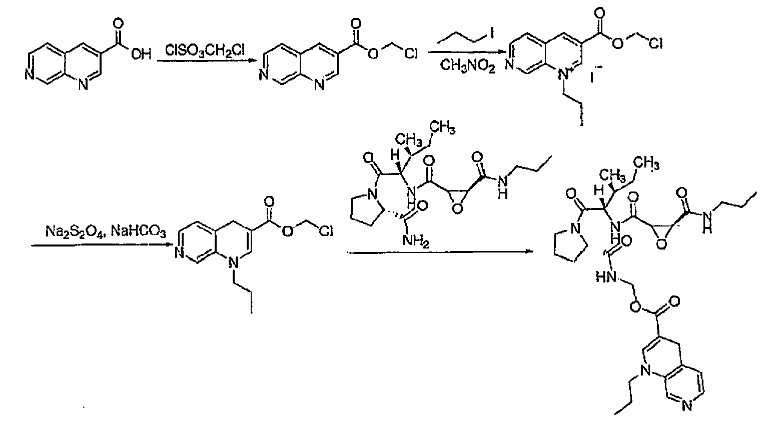

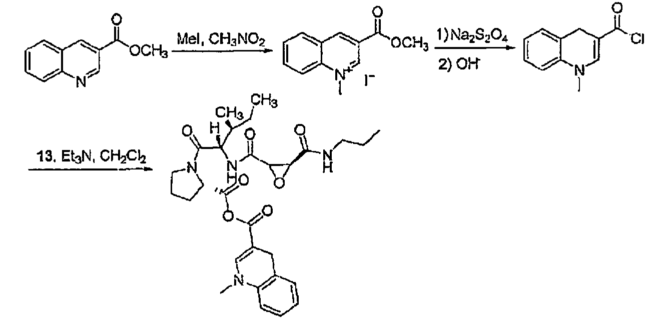

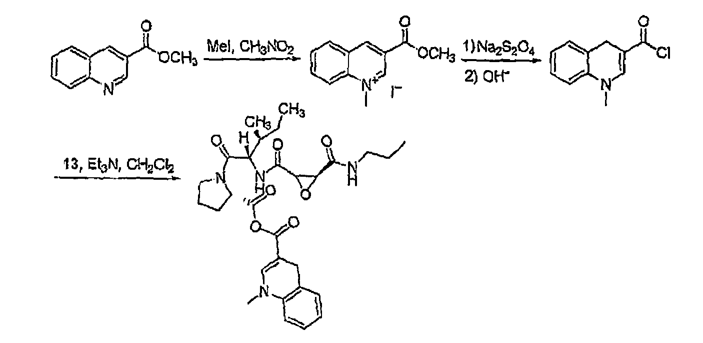

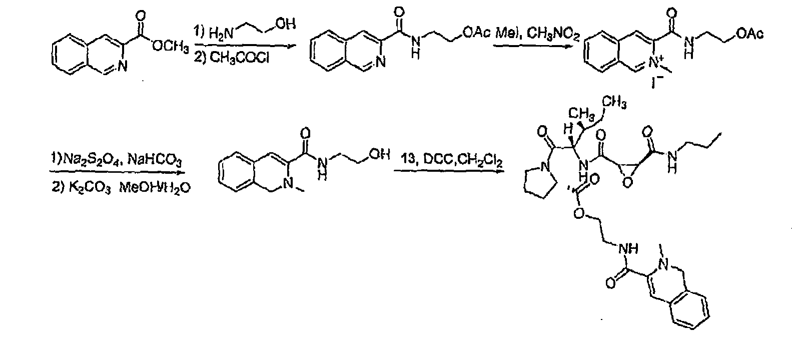

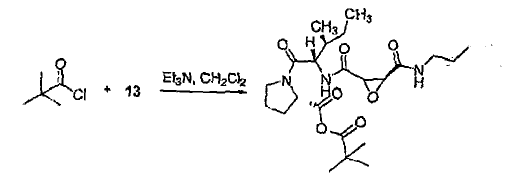

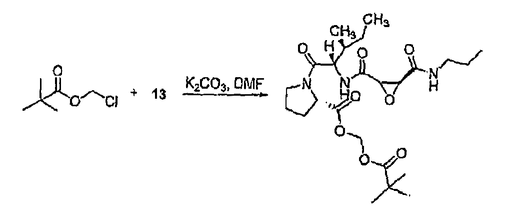

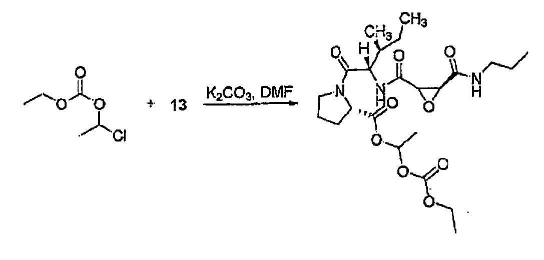

### EXAMPLE 4

As shown in Figure 1A, *in vivo* administration E64d to guinea pigs reduces brain Aβ by 31% and 55% relative to controls after dosing for 7 and 30 days, respectively Figure 1B shows that CA074Me reduces brain Aβ by 57% and 62% relative to controls after 7 and 30 days, respectively. Thus, the effectiveness of cysteine protease and cathepsin B inhibitors for reducing brain Aβ is validated and, as such, are effective AD therapeutics.

The following procedures are those of used in obtaining the data of the Examples 4-8.
*In vivo* icv administration of protease inhibitors into guinea pig brains. Guinea pigs (Harley strain, 300-500 grams) were obtained from Charles River (Wilmington, MA). The animals were given free access to food and water before and during the experiment. An ALZET osmotic mini-pump (ALZET company, Cupertino, CA) was implanted subcutaneously in the mid-scapular area of the back of each animal.. A catheter was connected to the osmotic mini-pump and inserted into the lateral ventricle of the brain (using a brain infusion kit manufactured by ALZET).

E64d or CA074Me (from CalBiochem, San Diego, CA) were each administered to guinea pig brains by icv minipump infusion at a rate of 2.5 ml/hr with each compound at 1 mg/ml in saline (with 1 5% DMSO). Each e64d and control group consisted of five animals; each CA074Me and control group consisted of ten animals. Continuous infusion was conducted for 7 days and 30 days.. At the end of each infusion period, animals were sacrificed and half the brain was prepared as extracts for ELISA measurement of Aβ peptide levels conducted in triplicate with Ab ELISAs (from Blosource international) The other half brain was used for isolation of synaptosome preparations and Ab analyses.
Brain Extracts. Brain extracts were prepared by subjecting fresh tissues to homogenization at 1:10 w/v in guanidine buffer consisting of 5 M guanidine-HCl in 50 mM Tris-HCl, pH 8.3 (buffer reagents from Sigma-Aldrich, St. Louis, MO). Homogenates were agitated at room temperature for 3-4 hours, then stored at -20°C The day before the ELISA assay, the tissue samples were diluted further by 1:10 for a final guanidine concentration of 0.5 M. These diluted homogenates were then centrifuged at 14,000 rpm for 20 minutes at 4°C and supernatants were collected for measurement of total Ab, and Aβ40 and Aβ42 separately, by ELISA assay.
Isolation of synaptosomes from brain. Synaptosomes were prepared by homogenization of fresh brain tissue in isotonic sucrose (320 mM), pH 7 4, 1 mM EDTA, 0 25 mM dithiothreitol, and 30 U/ml RNAse inhibitor. The homogenate was centrifuged at 1000 x g for 10 min. The supernatant was loaded on a Sucrose-Percoll discontinuous gradient and then centrifuged at 32,000 x g for 5 min. Enriched synaptosomes were washed in PBS, and centrifuged at 12,000 x g for 4 min. The supernatant was carefully removed, and the synaptosome-containing pellet was resuspended in 50% OptiPrep (Accurate Chemical, Westbury, NY), loaded to an OptiPrep discontinuous flotation gradient (9, 12.5, 25, and 35%) and centrifuged at 10,000 x g for 20 min. The synaptosomes were obtained at the 15-25% interface Synaptosomes were washed in PBS and then extracted as described for brain extracts.
Aβ analyses by ELISA assays. For quantitation of Aβ peptides, ELISA assays were used to measure Aβ levels.. For measurement of total Aβ, consisting of Aβ40 and Aβ42, ELISA assay was used (88-344/88-348 from Biosource International, Camarillo, CA). For measurement of Aβ40 and Aβ42, specific EL ISA assays were used respectively (88-348 and 88-344 from Biosource international), Brain and synaptosome extracts were subjected to ELISA assays as samples diluted in 0.5 M guanidine
Analyses of CTFβ and sAPPa fragments. CTFβ fragments derived from APP were analyzed by western blots with antibody MAB 1560 (Chemicon, Temecula, California). The sAPPα fragment derived from APP was analyzed by western blots with antibody MAB 343 (Chemicon, Temecula, California) Relative amounts of CTFb and sAPPa bands on western blots were achieved densitometry.
Data Analyses. Data were analyzed by one-way analysis of variance for analyses of statistical significance of the effects of compounds on Ab levels, with p < 0.05 indicating statistical significance, using GraphPad Prism software program Following ANOVA analyses among treatment groups, individual group values for Ab levels were analyzed using Dunnett's multiple comparison test. To facilitate comparisons among all studies, data are presented as percent of the corresponding vehicle control ± standard error of the mean (SEM).

### EXAMPLE 5

In Figure 2A and B, CA074Me causes approximately the same reduction in Aβ40 and Aβ42 relative to controls after 7 days of administration The fact that both forms are reduced equally is consistent with a cathepsin B inhibitor affecting the β-secretase activity, and probably cathepsin B β-secretase activity β-secretase activity produces both forms and, therefore, β-secretase inhibition would be expected to reduce both forms equally

### EXAMPLE 6

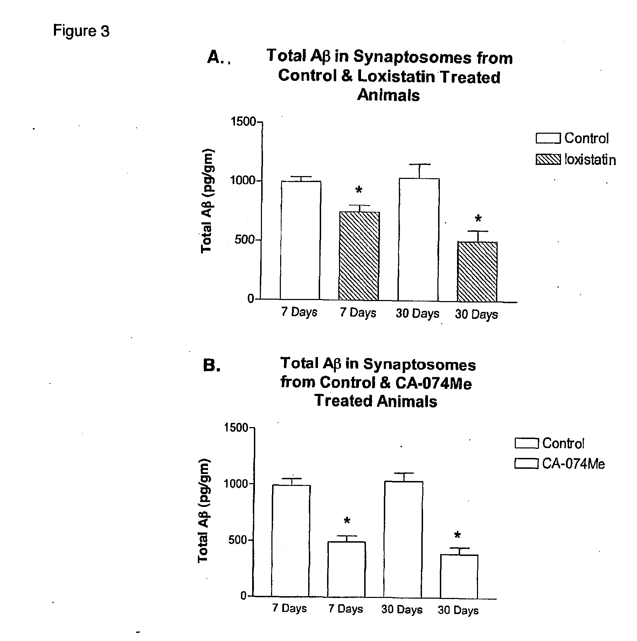

Figure 3A shows that e64d reduces synaptosome Aβ by 27% and 50% relative to controls after 7 and 30 days of administration, respectively. Figure 3B shows that CA074Me also reduces synaptosome Aβ by 62% and 74% after 7 and 30 days of administration respectively. Synaptosome Aβ is a measure of the Aβ produced by the neuronal regulated secretory pathway, the pathway by which most brain Aβ is produced (see for example, Verhage, McMahon, Ghijsen, Boomsma, Scholten, Wiegant and Nicholls, Differential release of amino acids, neuropeptides, and catecholamines from isolated nerve terminals, Neuron, 6(4):517-24,1991, Verhage, Ghijsen, Nicholls and Wiegant, Characterization of the release of cholecystokinin-8 from isolated nerve terminals and comparison with exocytosis of classical transmitters, J Neurochem, 56(4): 1394-400,1991, Nitsch, Farber, Growdon and Wurtman; Release of amyloid betaprotein precursor derivatives by electrical depolarization of rat hippocampal slices, Proc Natl Acad Sci U S A, 90(11):5191-3, 1993, Farber, Nitsch, Schulz and Wurtman, Regulated secretion of beta-amyloid precursor protein in rat brain, J Neurosci, 15(11):7442-51, 1995, Cirrito, Yamada, Finn, Sloviter, Bales, May, Schoepp, Paul, Mennerick and Holtzman, Synaptic Activity Regulates Interstitial Fluid Amyloid-β Levels In Vivo, Neuron, 48(913-922, 2005). Thus, the fact that a cathepsin B inhibitor reduces synaptosome Aβ shows that the inhibitor is affecting regulated secretion of Aβ and is consistent with cathepsin B acting in the regulated secretory pathway of neurons

### EXAMPLE 7

GTFβ is a proteolytic product of β-secretase cleavage of APP and is direct measure of β-secretase activity. Figure 4A shows CTFβ immunoblots and shows 7 days of CA074Me administration qualitatively reduces CTFβ relative to controls. Figure 4B shows the quantitative results from densometric scanning of the immunoblots and that CTFβ is 45% reduced after 7 days of CA074Me relative to controls This data is consistent with a cathepsin B inhibitor acting by affecting β-secretase activity.

### EXAMPLE 8

sAPPα is a proteolytic of α-secretase cleavage of APP The α-secretase cleaves APP with the Aβ sequence and thereby precludes Aβ formation, α-secretase and β-secretase can be, therefore, seen as competing for the same APP substrate.. Figure 5A shows sAPPα immunoblots and shows 7 days of CA074Me administration qualitatively increases sAPPα relative to controls Figure 5B shows the quantitative results from densometric scanning of the immunoblots and that sAPPα is 60% increased after 7 days of CA074Me relative to controls. The increase is consistent with the availability of more APP substrate due to decreased β-secretase activity.

Although the invention has been described with references to the examples provided above, modifications can be made without departing from the invention. Accordingly, the invention is limited only by the claims.

## Claims

1. A compound which, when metabolized, releases a cysteine protease inhibitor moiety, said compound having the structure: 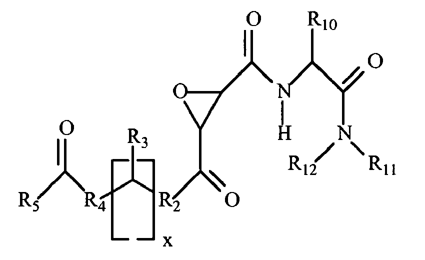 where R₂ is an O, NH, or N substituted with a C₁₋₆ alkyl group, optionally containing one or more aryl groups or heteroatoms;
X is 0, 1 or 2;
R₃, is absent when X is 0 and, when X is 1 or 2, R₃ is H, a C₁₋₆ alkyl group, optionally containing one or more aryl groups or heteroatoms, or an aryl group;
R₄ is absent when X is 0, or, when X is 1 or 2, R₄ is O, NH, or N substituted with a C₁₋₆ alkyl group, optionally containing one or more aryl groups or heteroatoms, or an aryl group; and
R₅ is a dihydropyridine ring attached via a dihydropyridine ring carbon, where:
the dihydropyridine ring carbons are each optionally substituted by a C₁₋₆ alkyl group optionally containing one or more aryl groups or heteroatoms, or an aryl group, or,
taken together, form a 5, 6, or 7 member aromatic or acyclic fused ring optionally containing one or more heteroatoms; and
the dihydropyridine ring nitrogen is substituted by a C₁₋₆ alkyl group, which optionally contains one or more aryl groups or heteroatoms, or an aryl group; and
R₁₀ is selected from a C₁₋₆ alkyl group, optionally containing one or more aryl groups or heteroatoms, an aryl group or a phenol group, or forms a part of a leucine or isoleucine group in the compound,
R₁₁ is selected from a C₁₋₁₀ alkyl group, optionally containing one or more aryl groups, heteroatoms, a guadinium group, an imidizol amine group or a propionylamino-acid methyl ester group, and
R₁₂ is H.

2. The compound of claim 1, having the structure: 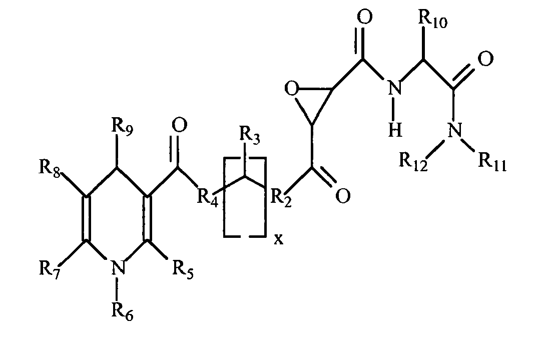 where:
R₅ is H, a C₁₋₆ alkyl group, optionally containing one or more aryl groups or heteroatoms, or an aryl group;
R₆ is a C₁₋₆ alkyl group, optionally containing one or more aryl groups, or heteroatoms, or an aryl group; and
R₇, R₈, and R₉ are each H, a C₁₋₆ alkyl group, optionally containing one or more aryl groups or heteroatoms, or an aryl group,, or, taken together, form a 5, 6, or 7 member aromatic or acyclic fused ring containing one or more heteroatoms.

3. The compound of claim 2, having the structure:

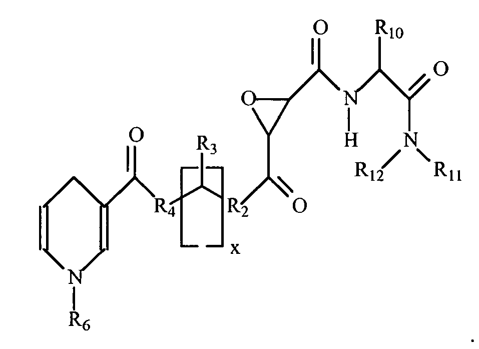

4. The compound of claim 2, having the structure:

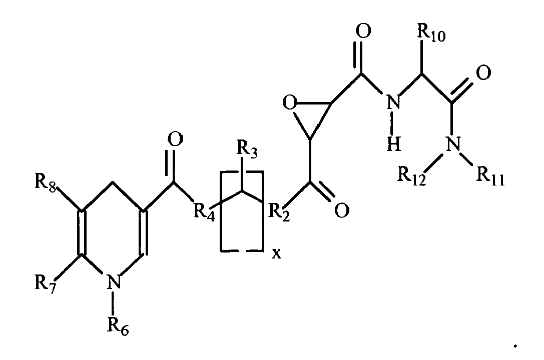

5. The compound of claim 2, having the structure:

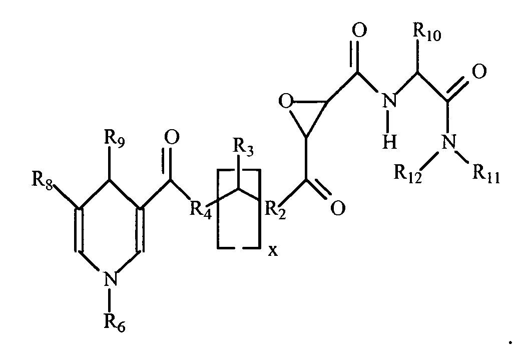

6. A compound which, when metabolized, releases a cysteine protease inhibitor moiety, said compound having the structure: 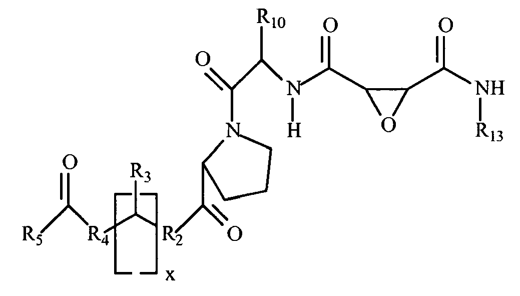 where: R₂ is O, NH, or N substituted with a C₁₋₆ alkyl group, optionally containing one or more aryl groups or heteroatoms;
X is 0, 1 or 2;
R₃, is absent when X is 0, or, when X is 1 or 2, R₃ is H, a C₁₋₆ alkyl group, optionally containing one or more aryl groups or heteroatoms, or an aryl group;
R₄ is absent when X is 0, or, when X is 1 or 2, R₄ is O, NH, or N substituted with a C₁₋₆ alkyl group, optionally containing one or more aryl groups or heteroatoms, or an aryl group; and
R₅ is a dihydropyridine ring attached via a dihydropyridine ring carbon, where:
the dihydropyridine ring carbons are each optionally substituted by a C₁₋₆ alkyl group optionally containing one or more aryl groups or heteroatoms, or an aryl group, or,
taken together, form a 5, 6, or 7 member aromatic or acyclic fused ring optionally containing one or more heteroatoms; and
the dihydropyridine ring nitrogen is substituted by a C₁₋₆ alkyl group, which optionally contains one or more aryl groups or heteroatoms, or an aryl group; and
R₁₃ is a C₁₋₆ alkyl group, optionally containing one or more aryl groups or heteroatoms, or an aryl group.

7. The compound of any one of claims 1 and 6, where R₅ is attached at an unsaturated dihydropyridine ring carbon.

8. The compound of claim 6, having the structure: 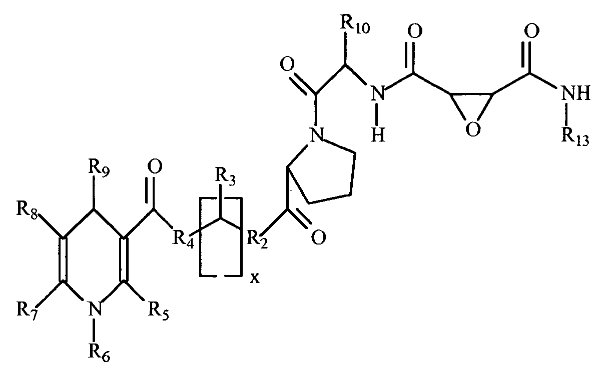 where:
R₅ is H, C₁₋₆ alkyl group, optionally containing one or more aryl groups or heteroatoms, or an aryl group;
R₆ is C₁₋₆ alkyl group, optionally containing one or more aryl groups or heteroatoms, or an aryl group; and
R₇, R₈, and R₉ are each H, C₁₋₆ alkyl group, optionally containing one or more aryl groups or heteroatoms, or an aryl group or, taken together, form a 5, 6, or 7 member aromatic or acyclic fused ring containing one or more heteroatoms.

9. The compound of claim 6, having the following structure:

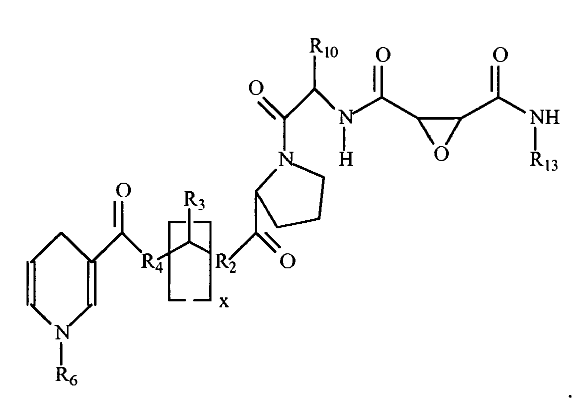

10. The compound of claim 8, having the structure:

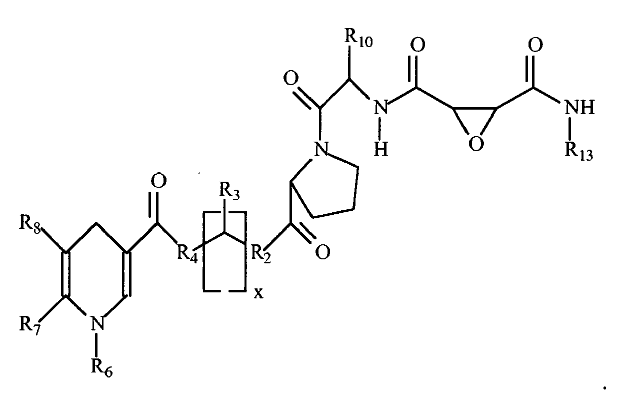

11. The compound of claim 8, having the structure:

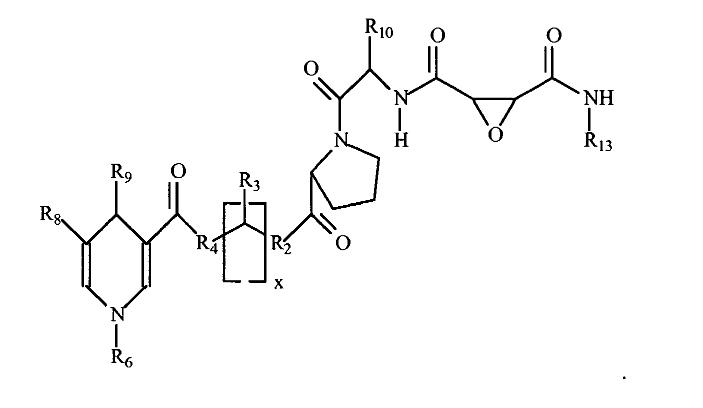

12. The compound of any one of claims 4, 5, 10 and 11, where R₈ and R₉ form a fused benzyl or pyridyl ring.

13. The compound of any one of claims 3, 9 and 12, where R₆ is methyl.

14. A pharmaceutical composition comprising the compound of any one of claims 1-13 and a pharmaceutically acceptable carrier.

15. A product as claimed in any one of claims 1-14, for use in treatment of dementia.

## Patentansprüche

1. Eine Verbindung, welche bei Metabolisierung eine Cystein-Protease-Inhibitor-Untereinheit freisetzt, wobei besagte Verbindung die folgende Struktur aufweist: 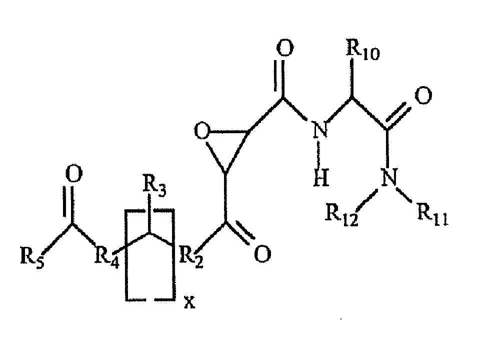 wobei R₂ ein O, NH oder N ist, substituiert mit einer C₁₋₆-Alkylgruppe, optional enthaltend eine oder mehr Arylgruppen oder Heteroatome;
X 0, 1 oder 2 ist;
R₃ abwesend ist, wenn X 0 ist, und, wenn X 1 oder 2 ist, R₃ H ist, eine C₁₋₆-Alkylgruppe, optional enthaltend eine oder mehr Arylgruppen oder Heteroatome, oder eine Arylgruppe;
R₄ abwesend ist, wenn X 0 ist, oder, wenn X 1 oder 2 ist, R₄ O, NH oder N ist, substituiert mit einer C₁₋₆-Alkylgruppe, optional enthaltend eine oder mehr Arylgruppen oder Heteroatome, oder eine Arylgruppe; und
R₅ ein über einen Dihydropyridin-Ring-Kohlenstoff gebundener Dihydropyridin-Ring ist, wobei: die Dihydropyridin-Ring-Kohlenstoffe jeweils optional durch eine C₁₋₆-Alkylgruppe substituiert sind, optional enthaltend eine oder mehr Arylgruppen oder Heteroatome, oder eine Arylgruppe, oder zusammengenommen einen 5-, 6- oder 7-gliedrigen aromatischen oder acyclischen kondensierten Ring bilden, optional enthaltend ein oder mehr Heteroatome; und
der Dihydropyridin-Ring-Stickstoff durch eine C₁₋₆-Alkylgruppe substituiert ist, optional enthaltend eine oder mehr Arylgruppen oder Heteroatome, oder eine Arylgruppe; und R₁₀ aus einer C₁₋₆-Alkylgruppe gewählt wird, optional enthaltend eine oder mehr Arylgruppen oder Heteroatome, eine Arylgruppe oder eine Phenolgruppe, oder Teil einer Leucin oder Isoleucin-Gruppe in der Verbindung bildet,
R₁₁ aus einer C₁₋₁₀-Alkylgruppe gewählt wird, optional enthaltend eine oder mehr Arylgruppen, Heteroatome, eine Guadinium-Gruppe, eine Imidizol-Amingruppe oder eine Propionyl-Aminosäure-Methylester-Gruppe, und
R₁₂ H ist.

2. Die Verbindung gemäß Anspruch 1 mit der folgenden Struktur: 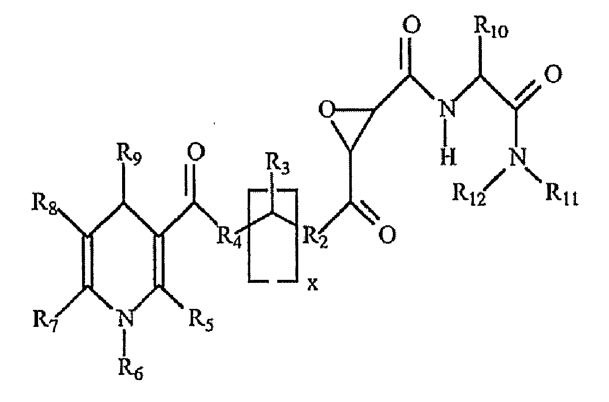 wobei:
R₅ H ist, eine C₁₋₆-Alkylgruppe, optional enthaltend eine oder mehr Arylgruppen oder Heteroatome, oder eine Arylgruppe;
R₆ eine C₁₋₆-Alkylgruppe ist, optional enthaltend eine oder mehr Arylgruppen oder Heteroatome, oder eine Arylgruppe; und
R₇, R₈ und R₉ jeweils H sind, eine C₁₋₆-Alkylgruppe, optional enthaltend eine oder mehr Arylgruppen oder Heteroatome, oder eine Arylgruppe, oder zusammengenommen einen 5-, 6- oder 7-gliedrigen aromatischen oder acyclischen kondensierten Ring bilden, enthaltend ein oder mehr Heteroatome.

3. Die Verbindung gemäß Anspruch 2 mit der folgenden Struktur:

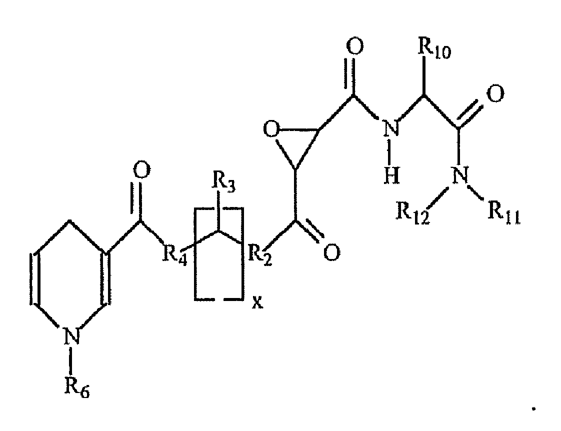

4. Die Verbindung gemäß Anspruch 2 mit der folgenden Struktur:

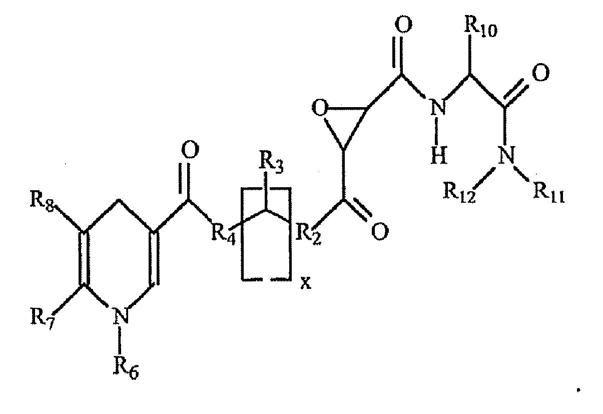

5. Die Verbindung gemäß Anspruch 2 mit der folgenden Struktur:

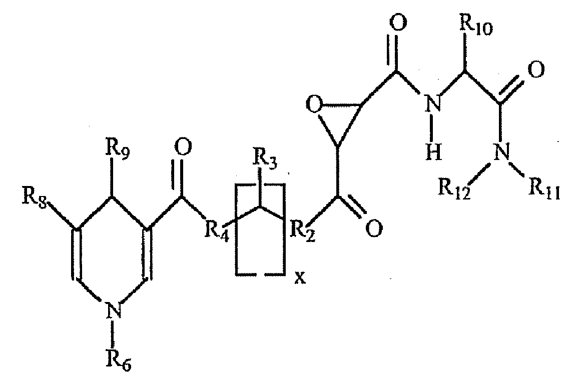

6. Eine Verbindung, die bei Metabolisierung eine Cystein-Protease-Inhibitor-Untereinheit freisetzt, wobei besagte Verbindung die folgende Struktur aufweist: 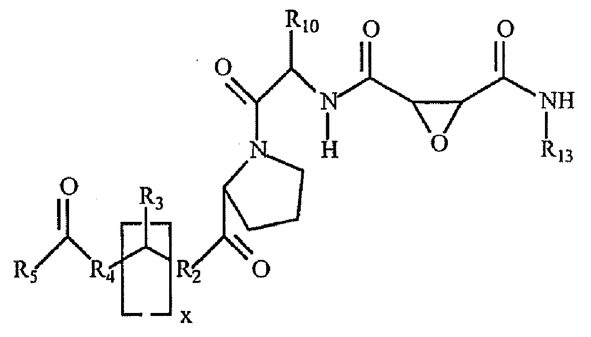 wobei: R₂ O, NH oder N ist, substituiert mit einer C₁₋₆-Alkylgruppe, optional enthaltend eine oder mehr Arylgruppen oder Heteroatome;
X 0, 1 oder 2 ist;
R₃ abwesend ist, wenn X 0 ist, oder, wenn X 1 oder 2 ist, R₃ H ist, eine C₁₋₆-Alkylgruppe, optional enthaltend eine oder mehr Arylgruppen oder Heteroatome, oder eine Arylgruppe;
R₄ abwesend ist, wenn X 0 ist, oder, wenn X 1 oder 2 ist, R₄ O, NH oder N ist, substituiert mit einer C₁₋₆-Alkylgruppe, optional enthaltend eine oder mehr Arylgruppen oder Heteroatome, oder eine Arylgruppe; und
R₅ ein über einen Dihydropyridin-Ring-Kohlenstoff gebundener Dihydropyridin-Ring ist, wobei: die Dihydropyridin-Ring-Kohlenstoffe jeweils optional durch eine C₁₋₆-Alkylgruppe substituiert sind, optional enthaltend eine oder mehr Arylgruppen oder Heteroatome, oder eine Arylgruppe, oder zusammengenommen einen 5-, 6- oder 7-gliedrigen aromatischen oder acyclischen kondensierten Ring bilden, optional enthaltend ein oder mehr Heteroatome; und
der Dihydropyridin-Ring-Stickstoff durch eine C₁₋₆-Alkylgruppe substituiert ist, optional enthaltend eine oder mehr Arylgruppen oder Heteroatome, oder eine Arylgruppe; und R₁₃ eine C₁₋₆-Alkylgruppe ist, optional enthaltend eine oder mehr Arylgruppen oder Heteroatome, oder eine Arylgruppe.

7. Die Verbindung gemäß irgendeinem der Ansprüche 1 und 6, wobei R₅ an einen ungesättigten Dihydropyridin-Ring-Kohlenstoff gebunden ist.

8. Die Verbindung gemäß Anspruch 6 mit der folgenden Struktur: 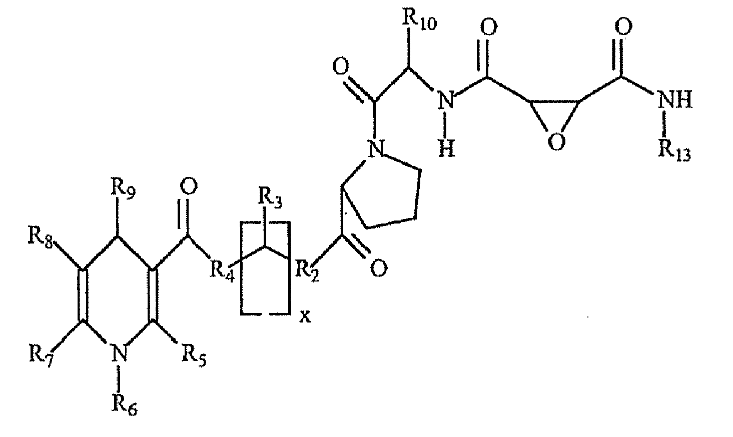 wobei:
R₅ H ist, C₁₋₆-Alkylgruppe, optional enthaltend eine oder mehr Arylgruppen oder Heteroatome, oder eine Arylgruppe;
R₆ C₁₋₆ Alkylgruppe ist, optional enthaltend eine oder mehr Arylgruppen oder Heteroatome, oder eine Arylgruppe; und
R₇, R₈ und R₉ jeweils H sind, C₁₋₆ Alkylgruppe, optional enthaltend eine oder mehr Arylgruppen oder Heteroatome, oder eine Arylgruppe, oder zusammengenommen einen 5-, 6- oder 7-gliedrigen aromatischen oder acyclischen kondensierten Ring bilden, enthaltend ein oder mehr Heteroatome.

9. Die Verbindung gemäß Anspruch 6 mit der folgenden Struktur:

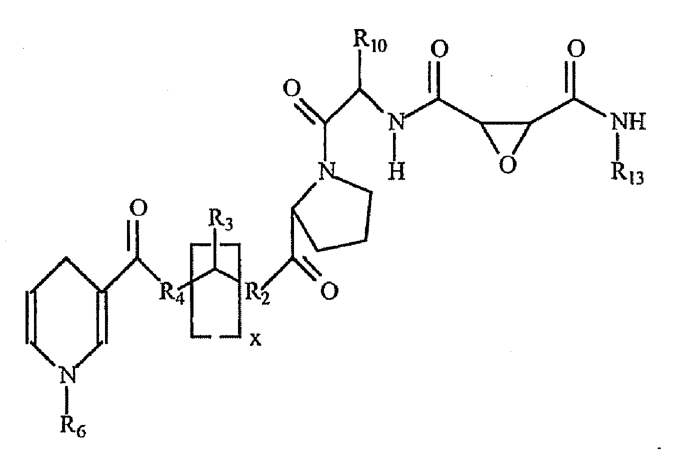

10. Die Verbindung gemäß Anspruch 8 mit der folgenden Struktur:

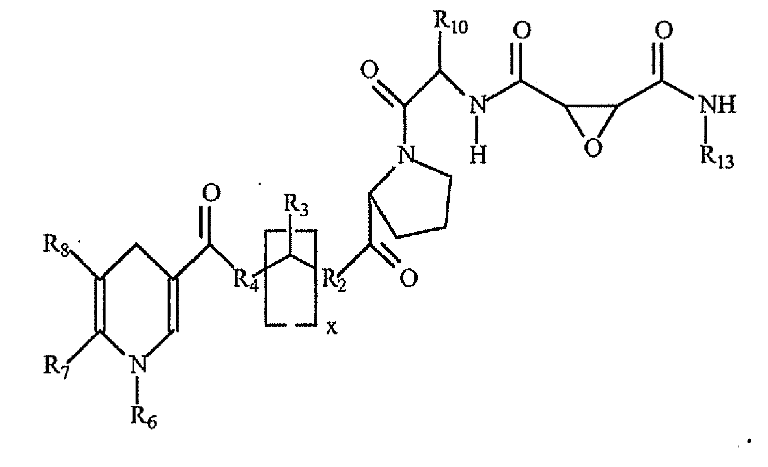

11. Die Verbindung gemäß Anspruch 8 mit der folgenden Struktur:

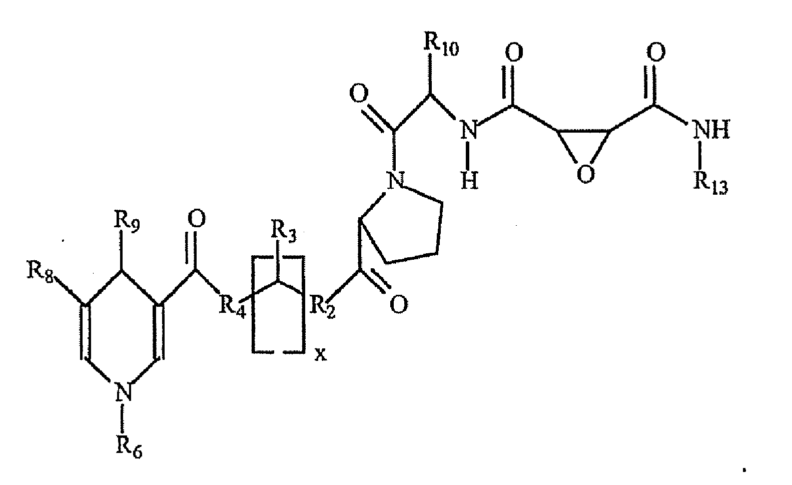

12. Die Verbindung gemäß irgendeinem der Ansprüche 4, 5, 10 und 11, wobei R₈ und R₉ einen kondensierten Benzyl- oder Pyridyl-Ring bilden.

13. Die Verbindung gemäß irgendeinem der Ansprüche 3, 9 und 12, wobei R₆ Methyl ist.

14. Eine pharmazeutische Zusammensetzung umfassend die Verbindung gemäß irgendeinem der Ansprüche 1 - 13 und ein pharmazeutisch akzeptabler Träger.

15. Ein Produkt gemäß irgendeinem der Ansprüche 1 - 14 zum Gebrauch bei der Demenzbehandlung.

## Revendications

1. Composé qui, lorsque métabolisé, libère une fraction d'inhibiteur de cystéine protéase, ledit composé ayant la structure : 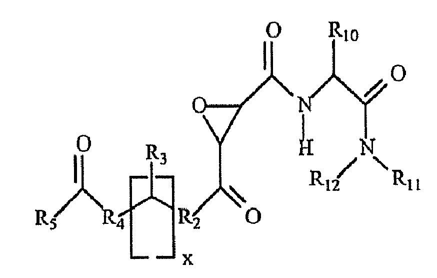 où R₂ représente un O, NH, ou N substitué par un groupe alkyle C₁₋₆, contenant éventuellement un ou plusieurs groupes aryles ou hétéroatomes;
X est égal à 0, 1 ou 2;
R₃ est absent lorsque X égal 0, et lorsque X est égal à 1 ou 2, R₃ représente un H, un groupe alkyle C₁₋₆, contenant éventuellement un ou plusieurs groupes aryles ou hétéroatomes, ou un groupe aryle ;
R₄ est absent lorsque X égal 0, ou lorsque X est égal à 1 ou 2, R₄ représente un O, NH ou N substitué par un groupe alkyle C₁₋₆, contenant éventuellement un ou plusieurs groupes aryles ou hétéroatomes, ou un groupe aryle ; et
R₅ représente un anneau de dihydropyridine rattaché via un carbone d'anneau de dihydropyridine, où : les carbones de l'anneau de dihydropyridine sont indépendamment éventuellement substitué par un groupe alkyle C₁₋₆, contenant éventuellement on ou plusieurs groupes aryles ou hétéroatomes, ou un groupe aryle; ou en combinaison forment un anneau aromatique ou acyclique soudé à 5, 6 ou 7 membres, contenant éventuellement un ou plusieurs hétéroatomes; et
l'azote de l'anneau de dihydropyridine est substitué par un groupe alkyle C₁₋₆, qui éventuellement contient un ou plusieurs groupes aryles ou hétéroatomes, ou un groupe aryle, et
R₁₀ est sélectionné à partir d'un groupe alkyle C₁₋₆, contenant éventuellement un ou plusieurs groupes aryles ou hétéroatomes, un groupe aryle, ou un groupe phénol, ou forme une partie d'un groupe leucine ou isoleucine du composé,
R₁₁ est sélectionné à partir d'un groupe alkyle C₁₋₁₀, contenant éventuellement un ou plusieurs groupes aryles, hétéroatomes, un groupe guadinium, un groupe amine imidizole ou un groupe acide aminopropionyl ester de méthyle , et
R₁₂ représente un H.

2. Composé de la revendication 1 ayant la structure : 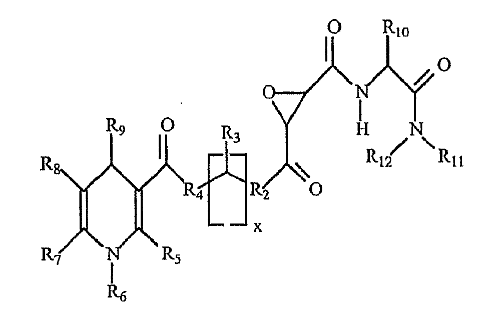 où:
R₅ représente un H, un groupe C₁₋₆ alkyle, contenant éventuellement un ou plusieurs groupes aryles ou hétéroatomes ou un groupe aryle;
R₆ représente un groupe alkyle C₁₋₆, contenant éventuellement un ou plusieurs groupes aryles, ou hétéroatomes, ou un groupe aryle, et
R₇, R₈, et R₉ représentent indépendamment un H, un groupe alkyle C₁₋₆, contenant éventuellement un ou plusieurs groupes aryles ou hétéroatomes, ou un groupe aryle, ou en combinaison forment un anneau aromatique ou acyclique soudé à 5, 6, ou 7 membres, contenant un ou plusieurs hétéroatomes.

3. Composé de la revendication 2 ayant la structure:

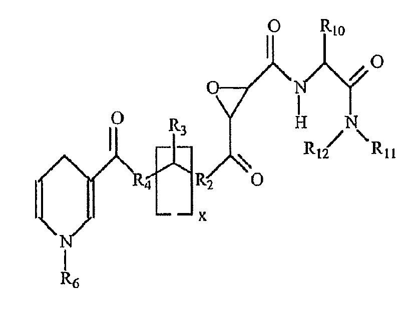

4. Composé de la revendication 2 ayant la structure:

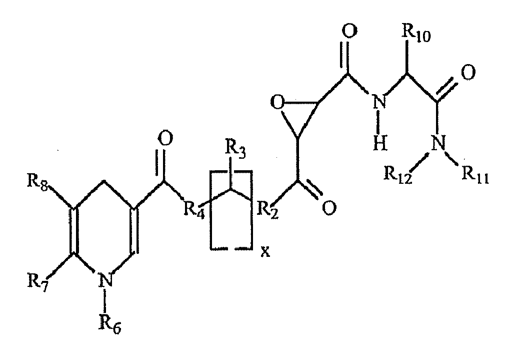

5. Composé de la revendication 2 ayant la structure:

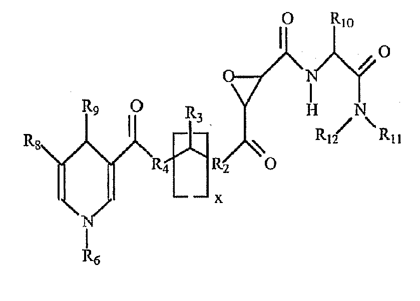

6. Composé qui lorsque métabolisé, libère une fraction d'inhibiteur de cystéine protéase, ledit composé ayant la structure : 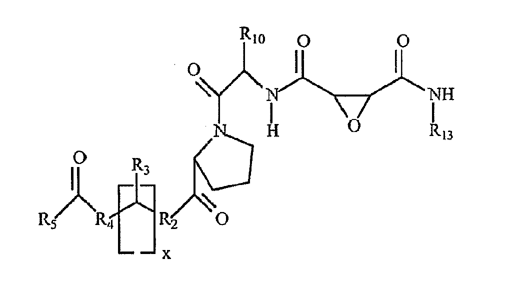 où R₂ représente un O, NH, ou N substitué par un groupe alkyle C₁₋₆, contenant éventuellement un ou plusieurs groupes aryles ou hétéroatomes;
X est égal à 0, 1 ou 2;
R₃ est absent lorsque X égal 0, ou lorsque X est égal à 1 ou 2, R₃ représente un H, un groupe alkyle C₁₋₆, contenant éventuellement un ou plusieurs groupes aryles ou hétéroatomes, ou un groupe aryle ;
R₄ est absent lorsque X égal 0, ou lorsque X est égal à 1 ou 2, R₄ représente un O, NH ou N substitué par un groupe alkyle C₁₋₆, contenant éventuellement un ou plusieurs groupes aryles ou hétéroatomes, ou un groupe aryle ; et
R₅ représente un anneau de dihydropyridine rattaché via un carbone d'anneau de dihydropyridine, où : les carbones de l'anneau de dihydropyridine sont indépendamment éventuellement substitué par un groupe alkyle C₁₋₆ contenant éventuellement un ou plusieurs groupes aryles ou hétéroatomes, ou un groupe aryle; ou en combinaison forment un anneau aromatique ou acyclique soudé à 5, 6 ou 7 membres contenant éventuellement un ou plusieurs hétéroatomes; et
l'azote de l'anneau de dihydropyridine est substitué par un groupe alkyle C₁₋₆, qui éventuellement contient un ou plusieurs groupes aryles ou hétéroatomes, ou un groupe aryle, et
R₁₃ représente un groupe alkyle C₁₋₆, contenant éventuellement un ou plusieurs groupes aryles ou hétéroatomes, ou un groupe aryle.

7. Composé de l'une quelconque des revendications 1 à 6, où R₅ est rattaché à un carbone de l'anneau dihydropyridine non saturé.

8. Composé de la revendication 6 ayant la structure : 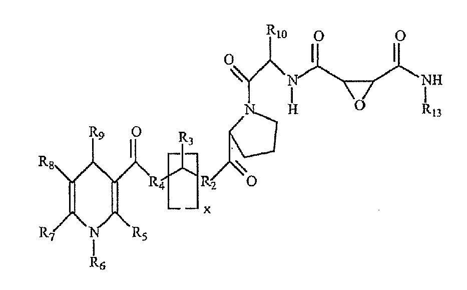 où:
R₅ représente un H, un groupe C₁₋₆ alkyle, contenant éventuellement un ou plusieurs groupes aryles ou hétéroatomes ou un groupe aryle;
R₆ représente un groupe alkyle C₁₋₆, contenant en option un ou plusieurs groupes aryles, ou hétéroatomes, ou un groupe aryle, et
R₇, R₈, et R₉ représentent indépendamment un H, un groupe alkyle C₁₋₆, contenant éventuellement un ou plusieurs groupes aryles ou hétéroatomes, ou un groupe aryle, ou en combinaison forment un anneau aromatique ou acyclique soudé à 5, 6, ou 7 membres, contenant un ou plusieurs hétéroatomes.

9. Composé de la revendication 6 ayant la structure:

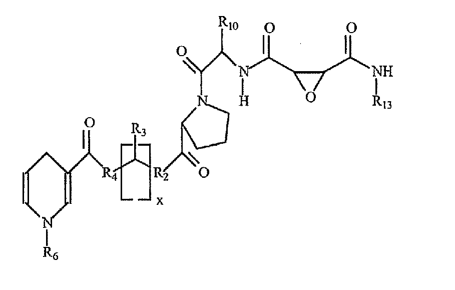

10. Composé de la revendication 8 ayant la structure:

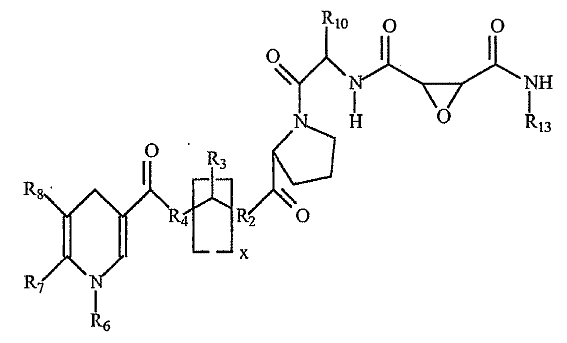

11. Composé de la revendication 8 ayant la structure:

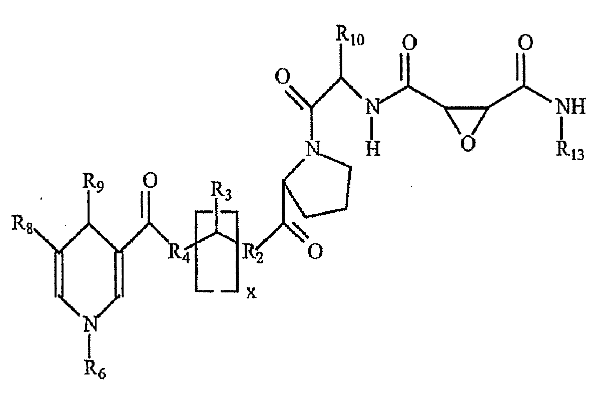

12. Composé de l'une quelconque des revendications 4, 5, 10 et 11, où R₈ et R₉ forment un anneau soudé de benzyle ou de pyridyle.

13. Composé de l'une quelconque des revendications 3, 9 et 12, où R₆ représente un méthyle.

14. Composition pharmaceutique comprenant le composé de l'une quelconque des revendications 1 à 13 et un support acceptable.

15. Produit revendiqué selon l'une quelconque des revendications 1 à 14, pour utilisation destinée au traitement de la démence.
